Europäisches Patentamt

European Patent Office

Office européen des brevets

(11) Veröffentlichungsnummer: **0 179 108**
**B1**

(12) **EUROPÄISCHE PATENTSCHRIFT**

(45) Veröffentlichungstag der Patentschrift:
12.07.89

(21) Anmeldenummer: **85902022.4**

(22) Anmeldetag: **17.04.85**

(86) Internationale Anmeldenummer:
**PCT/EP 85/00170**

(87) Internationale Veröffentlichungsnummer:
**WO 85/04957 (07.11.85 Gazette 85/24)**

(51) Int. Cl.⁴: **G 01 N 33/10,** G 01 N 21/47

(54) **INFRAROT MESSVORRICHTUNG ZUR KONTINUIERLICHEN UNTERSUCHUNG VON MAHLGÜTERN.**

(30) Priorität: **19.04.84 CH 1964/84**
**08.05.84 CH 2236/84**
**15.11.84 DE 3441856**

(43) Veröffentlichungstag der Anmeldung:
**30.04.86 Patentblatt 86/18**

(45) Bekanntmachung des Hinweises auf die Patenterteilung:
**12.07.89 Patentblatt 89/28**

(84) Benannte Vertragsstaaten:
**AT BE CH DE FR GB IT LI LU NL SE**

(56) Entgegenhaltungen:
**EP-A-0 061 437**
**FR-A-2 437 621**
**US-A-3 321 636**
**US-A-4 422 760**

(73) Patentinhaber: **Gebrüder Bühler AG, CH- 9240 Uzwil (CH)**

(72) Erfinder: **BISCHOFF, Bruno, Sturzeneggerstrasse 19, CH- 9015 St. Gallen (CH)**

(74) Vertreter: **EGLI- EUROPEAN PATENT ATTORNEYS, Widenmayerstrasse 5, D-8000 München 22 (DE)**

LIBER, STOCKHOLM 1989

## Beschreibung

Die Erfindung bezieht sich auf eine Infrarot-Meßvorrichtung für die kontinuierliche, quantitative Bestimmung einzelner Bestandteile von Mehl oder anderen Nahrungsmittel-Mahlgütern, in welcher das Meßgut durch eine Meßstrecke geführt und dort im Bereich eines Meßaufnehmers verdichtet wird.

In der getreideverarbeitenden Industrie wird schon seit einiger Zeit die Infrarotspektroskopie zur Messung verschiedener Inhaltsstoffe (wie Protein und Wasser) in Mehl angewendet. Diese Inhaltsstoffe weisen unter Infrarotlicht ein ganz typisches Lichtabsorbtions- und Reflexionsverhalten auf.

Aufgrund natürlicher Faktoren von Boden und Klima und Klimastabilität sind insbesondere z. B. im europäischen und angrenzenden Raume sehr unterschiedliche Getreideernten eine Tatsache. Ebenfalls im Hinblick auf die Getreideerzeugung sind Räume wie USA, Kanada und Australien zusätzlich privilegiert, indem nicht nur weniger Klimaschwankungen eintreten, sondern darüberhinaus erlauben Boden und Klima die besten Qualitäten des Getreides zu produzieren, soweit man die größeren Mengen des internationalen Getreidehandels in Betracht zieht. Auch hier gilt, wie bei vielen anderen Gütern, daß für höhere Qualität auch höhere Preise bezahlt werden müssen. Hinzu kommt, daß jedes Land versucht, die größtmögliche Erzeugung an Landwirtschaftsprodukten im eigenen Land zu erhalten. Der Konsument verlangt Brotprodukte, die seinen Vorstellungen entsprechen, sei es Geschmack, Luftigkeit, Nährwert, Preis, usw. Das Zuviel und Zuwenig wird durch den internationalen Handel ausgeglichen, jedoch mit dem Nachteil von stark variierenden Qualitäten der Getreideinhaltsstoffe. Mühle und Bäckerei (mit Berücksichtigung der staatlichen Interessen) bleiben die Optimierungsaufgaben, um mit

- dem größtmöglichen Anteil an billigem Getreide (tiefe Proteinwerte usw.) und
- dem kleinstmöglichen Anteil an teurem Getreide (höhere Proteinwerte usw.)

das bestmögliche Mehl bzw. Brot herzustellen.

Heute hat der Markt die Eigenschaft, daß Angebot und Preis starken und schnellen Änderungen unterworfen sind, so daß es für eine Mühle heute nicht mehr tragbar ist, daß sozusagen nach alten Erfahrungswerten Getreide gemischt und Wasser zugegeben wird. Der Markt und speziell der buchhalterische Gesichtspunkt verlangen eine dauernde Anpassung an die jeweils vorliegenden Bedingungen, was nach dem Einsatz von Computern ruft.

Größere Nachforschungen, insbesondere aber eine labormäßige Überprüfung der besten zur Zeit bekannten Infrarotmeßgeräte führten zu dem Schluß, daß wohl die Labormessung, nicht aber kontinuierliche Messungen in der Fabrikation als gelöst angesehen werden können.

Die Handhabung von Labormeßwerten ist in einer Hinsicht sehr einfach: weicht das Resultat von der Realität ab, wird die Messung im eigenen oder in einem fremden Labor wiederholt. Man kann aber auch das Labormeßergebnis ignorieren und weiterfabrizieren, wenn alle anderen Werte (auch die durch eine sensorische Beurteilung gewonnenen) dies als verantwortbar erscheinen lassen. Heir steht der Mensch mit seinem Entscheid dazwischen.

Bei der Untersuchung des Problemkreises hatten sich bisher drei "Barrieren" gezeigt, die als ungelöst gelten:

1) Eine Labormeßeinrichtung kann bezüglich ihrer Bausteine (Elektronik usw.) für weniger große Umwelteinflüsse konzipiert sein. Wird ein Labormeßgerät jedoch im Fabrikationsbetrieb eingesetzt, sind die Fehler, die durch die Umwelteinflüsse bedingt sind, in vielen Fällen von anderen Störungen nicht trennbar.

2) Ist ein neues System, hier die Infrarotspektroskopie, von der theoretischen, besonders der physikalischen, chemischen und mathematischen Seite her erforscht, im Labor überprüft und wird das als brauchbar erkannte System der Einsatzwirklichkeit unterworfen, so zeigt sich häufig zunächst Unbrauchbarkeit im Hinblick auf die praktische Eignung (d.h. durch nichts erklärbare Fehler und Abweichungen treten auf).

3) Auch für größere Produktmengen repräsentative Aussagen lassen sich aufgrund praktischer Messungen bei Einzelproben vielfach nicht ausreichend sicher ableiten.

Die eingangs genannte gattungsgemäße Infrarot-Meßvorrichtung ist aus der EP-A-0 061 437 (PERTEN) bekannt. Mit dieser Meßvorrichtung soll insbesondere Mehl auf seinen Gehalt an Protein, Wasser und Fett untersucht werden: Die EP-A-0 061 437 beschreibt im wesentlichen drei Arten von Meßvorrichtungen und damit durchzuführende Meßverfahren. Im einen Fall handelt es sich offenbar um ein reines Labormeßgerät, daß sich nicht zur kontinuierlichen Meßung der Bestandteile in der laufenden Produktion eignet. In diesem Fall wird die Meßprobe in einen oben offenen, sonst aber geschlossenen Behälter gefüllt und mit einem Verdichter in einer parallel zum Meßaufnehmerfenster liegenden Richtung verdichtet.

In den beiden anderen Fällen ist offenbar der Einsatz der Meßvorrichtung im Rahmen der laufenden Produktion angesprochen. Im einen Fall wird hierbei das Meßgut durch einen im wesentlichen vertikal verlaufenden By-Pass geleitet. Der By-Pass ist an seinem unteren Ende mittels einer Absperrplatte verschließbar. Hierdurch wird das Gut durch sein Eigengewicht verdichtet, und zwar wiederum in einer Richtung, die parallel zur Meßaufnehmerfläche verläuft. Im anderen Fall ist zur Verdichtung offenbar eine Zwangsförderung vorgesehen, so daß das Meßgut während der Meßphase am Meßaufnehmer in Richtung der Zwangsförderung vorbeistreicht. Es tritt also während der Dauer der Messung eine kontinuierliche Bewegung in dem zu vermessenen Mahlgut auf, wodurch sich laufend Veränderungen in der Oberflächenstruktur, die dem Meßlicht ausgesetzt sind, ergeben. Die Genauigkeit, der mit einem Infrarot-Reflexionsmeßgerät gewonnen Meßergebnisse ist aber wesentlich von der Oberflächenstruktur bestimmt, auf welche das Infrarot-Licht fällt und von der es dann in den Meßaufnehmer reflektiert wird. Bei dem bekannten Verfahren verändert sich zum einen die örtliche Lage der einzelnen Gutkörper innerhalb des Bereiches des auftreffenden Meßlichtes. Zum anderen bilden sich durch die Bewegung im leicht verdichteten Gut bestimmte Strömungstrukturierungen aus, die sich aus einer komplizierten Wechselwirkung zwischen Wandreibung an dan Wänden der Meßstrecke und andererseits aus der Druckverteilung sowie dem Strömungsbild innerhalb des komprimierten Meßgut-Stranges in der rohrförmigen Meßstrecke ergeben. Die dabei während der Meßung auftretenden zeitabhängigen laufenden Veränderungen in der zu vermessenden Meßgut-Oberfläche führen zu Ungenauigkeiten bei den davon abgeleiteten Meßwerten.

Bei den zusätzlich in der EP-A-0 061 437 beschriebenen Fällen zur kontinuierlichen Messung, jedoch ohne Zwangsförderung, ruht das Gut während der Messung und wird nur einmal verdichtet, und zwar stets in einer zur Meßaufnehmeroberfläche parallelen Richtung. Auch hierdurch können sich infolge der oben angegebenen Wandreibung beim Verdichten unerwünschte Oberflächenstrukturen der Meßprobe ergeben.

Die FR-A-437 621 (GEBRÜDER BÜHLER AG) beschreibt eine Vorrichtung zur Helligkeitsmessung und zur Kontrolle des Homogenitätsgrades von Mehl, ohne daß jedoch die Bestimmung der Inhaltsstoffe des Mehles angesprochen würde, erst recht nicht die Bestimmung derartiger Inhaltsstoffe mittels Infrarotlicht.

Der Erfindung liegt die Aufgabe zugrunde, eine Vorrichtung und ein Verfahren zur kontinuierlichen Bestimmung einzelner Bestandteile von Mehl oder anderen Nahrungsmittel-Mahlgütern zu entwickeln, welche die beschriebenen Nachteile vermeiden und insbesondere zu repräsentativen Meßresultaten führen, die bei einer Anwendung in der Praxis derart zuverlässig sind, daß damit die entsprechenden Parameter in der Verarbeitung direkt steuer- bzw. regelbar sind. Im Vordergrund steht dabei die Bestimmung der Proteinwerte sowie des Wassergehalts bei Mahlgütern (wie insbesondere Mehl und mehlige Güter).

Eine Nebenaufgabe liegt aber auch in der Möglichkeit zur gleichzeitigen Erfassung weiterer Parameter, wie z. B. Asche und Farbe des Mahlgutes.

Diese Aufgabe wird dadurch gelöst, daß bei der eingangs genannten gattungsgemäßen Infrarot-Meßvorrichtung Mittel zum leichten Pressen und Glätten des Mahlgutes in der Meßstrecke vorgesehen sind; ferner eine Einrichtung zum zusätzlichen Verdichten des leicht gepreßten und geglätteten Meßgutes in senkrechter Richtung gegen den Meßaufnehmer und schließlich Mittel zum Stoppen der Bewegung des so verdichteten Meßgutes und Auslösen der eigentlichen Infrarot-Meßphase.

Es hat sich überraschend gezeigt, daß die Erfindung die aufgezeigten "Barrieren" völlig entfallen läßt und die dabei auftretenden Probleme überwindet.

Bei der Erfindung wird das Produkt in der Meßstrecke in leicht vorgepreßten Zustand gebracht und dann im Bereich des Meßaufnehmers für die Infrarotmessung verdichtet. Die Vorbereitung wird damit bewußt in zwei Schritten durchgeführt. Zuerst wird das Produkt unter leichten Druck gesetzt, so daß mit Sicherheit keine Produkthohlräume mehr vorhanden sind und das Mehl ständig glatt an dem Meßaufnehmer anliegt, sogar leicht dagegen gepreßt wird. Zusätzlich wird das Meßgut während der Messung stationär gehalten. Damit aber sind konstante Arbeitsbedingungen geschaffen. Ein Schüttgut unterliegt bekanntlich wesentlich anderen Gesetzen als eine Flüssigkeit. Beim Schüttgut ist die gegenseitige Abstützung der Partikel ein hervortretendes Charakteristikum. Das durch die Zwangsförderung leicht gepreßte Mehl wird sinngemäß zu einer Büchse, wie sie häufig in einer Laboreinrichtung für die Messung von Materialeigenschaften verwendet wird. Im Zwangsförderraum kann das Mehl nicht "fliehen". Also läßt sich örtlich in dem Mehl eine Verdichtung herstellen. Die Verdichtung wird im Bereich des Meßaufnehmers erzeugt. Störende "Randbedingungen" fallen weg, da die Verdichtung innerhalb desselben leicht gepreßten Mehles erzeugt wird und die Luft entweichen kann. Die Meßprobe und die Meßbedingungen sind in jeder Hinsicht optimal und reproduzierbar. Somit kann, wie bereits ausgeführt, die der Erfindung zugrundeliegende Aufgabe auf überraschend vorteilhafte Weise realisiert werden.

Die Erfindung erlaubt nun verschiedene besonders vorteilhafte Weiterausgestaltungen. Bevorzugt weist das Infrarotmeßgerät eine Zwangsförderung zum Vorverdichten des Meßgutes und eine Steuereinrichtung zur Unterbrechung der Zwangsförderung während einer Infrarotmeßphase auf. Mit der Unterbrechung der Zwangsförderung kann ein stetiger Meßgutdurchfluß sichergestellt werden. Die ganze Meßgutsäule ruht während der Meßzeit, z. B. für 3 bis 30 Sekunden. Nach Beendigung der Messung wird die ganze Produktmenge also mit der verdichteten Meßprobe weggefördert. Neues Produkt fließt nach, so daß die Gefahr einer wiederholten Messung der gleichen Probe (etwa weil diese im Bereich des Meßaufnehmers haften blieb) ausgeschaltet ist. Die Zwangsförderung trägt zu einem problemfreien Funktionieren vorzüglich bei. Mit dem leichten Verschiebedruck, der aufgewendet werden muß, wird auch die Meßprobe weggeschoben. Wie noch auszuführen ist, tritt eine zusätzliche Unterstützung ein, wenn die Zwangsförderung mit Vibration erfolgt: das verdichtete Mehl wird bei erneuter Zuschaltung der Vibration bzw. der Zwangsförderung wieder gelockert. Vorteilhaft ist es, wenn unabhängige Zeiteinstellelemente für die Unterbrechung der Zwangsförderung für die zyklische Verdichtung des Meßgutes und für das Auslösen sowie Steuern der Infrarotmeßphase vorgesehen sind. Damit läßt sich die Meßvorbereitung optimal gestalten und die Meßprobe für die Infrarotmessung beruhigen. Die Meßstrecke kann vorteilhaft im Bereich des Meßaufnehmers eine rohrförmig geschlossene Form aufweisen, derart, daß ein Meßrohr gebildet wird. Das Infrarotmeßgerät sollte an dem Meßrohr derart befestigt sein, daß die optische Achse des Infrarotmeßgerätes im wesentlichen senkrecht zur Meßgutoberfläche ausgerichtet ist. Die besten Resultate werden erzielt, wenn die das Meßgut senkrecht gegen den Meßaufnehmer verdichten den steuerbaren Druckmittel (Einrichtung zum zyklischen Verdichten des Meßgutes) an der Meßstrecke im Bereich des Meßaufnehmers, jedoch diesem gegenüberliegend angeordnet sind, wodurch zwischen dem Produkt und dem Meßaufnehmer die bestmögliche Zuordnung hergestellt wird. Dem Meßaufnehmer präsentiert sich eine klar definierte Meßfläche, welche zudem eine definierte Oberflächenbeschaffenheit besitzt.

Zweckmäßig werden die Druckmittel mit einem Druckkörper ausgebildet, der in Richtung des Meßaufnehmers verschiebbar und, wieder vorzugsweise, von einem weg- und druckeinstellbaren Pneumatikzylinder betätigbar ist. Der Druckkörper kann vorteilhafterweise aber auch Teil einer weg- und krafteinstellbaren Magnetspule sein.

Eine weitere vorzugsweise Ausgestaltung der Erfindung besteht auch darin, daß der Druckkörper als bewegbarer Löffel ausgebildet ist. Dabei wird vorzugsweise der Löffel verschwenkbar und über pneumatische oder elektrische Antriebsmittel gegen den Meßaufnehmer hin im wesentlichen in Richtung der optischen Achse bewegbar angeordnet. Erste Versuche mit einer Löffelform brachten sehr günstige Resultate. Bei diesen Versuchen war der Löffel wie ein Suppenlöffel ausgebildet und seine konkave Seite (Löffelinnenseite) dem Meßaufnehmer zugewendet, so daß das Meßgut mit dem Löffel gegen den Meßaufnehmer hin verdichtet wurde.

Eine vorteilhafte Weiterbildung der Erfindung besteht auch darin, daß die Einrichtung zum zyklischen Verdichten des Meßgutes aufblasbare Druckkissen aufweist, die an der Meßstrecke auf der dem Meßaufnehmer gegenüberliegenden Seite angeordnet sind. Das Druckkissen erstreckt sich vorzugsweise längs der Meßstrecke und weist eine Länge auf, die zumindest ein Mehrfaches der Längsabmessung der Meßfläche beträgt.

Besonders gute Meßresultate wurden bei Versuchen mit einer als Kolben ausgebildeten Einrichtung zum zyklischen Verdichten des Meßgutes erhalten, wobei der Kolben vorzugsweise pneumatisch oder elektrisch betätigt wird. Dabei ist weniger die Art der verwendeten Arbeitsmittel, ob Druckluft, Drucköl oder Elektrizität, sondern insbesondere die Steuerbarkeit von Weg, Zeit und Druck entscheidend.

Eine ganz besonders günstige Ausgestaltung der Erfindung besteht ferner darin, daß die Meßstrecke als Vibromeßstrecke ausgebildet und damit die Zwangsförderung durch die Vibration sicherstellbar ist.

Die Vibromeßstrecke wird besonders vorzugsweise als By-Pass zu einem Hauptproduktstrom ausgeführt und, wiederum vorzugsweise, der Vibrator glockenartig im inneren der Meßstrecke pendelnd aufgehängt. In der Meßstrecke bewirkt die Vibration gleichzeitig eine Zwangsförderung. Der Vibrator bildet am unteren Ende der Meßstrecke durch seine glockenartige Form und den entsprechenden Wandteil des Gehäuses einen Dosierspalt aus.

Vorteilhafterweise ordnet man die Meßstrecke im Überlaufprinzip und in einem By-Pass derart an, daß vom Hauptproduktstrom ein erster Teilstrom durch Schwerkraft in die Meßstrecke fließt und der Rest den Hauptstrom bildet. Die Dosierleistung der Vibrationsförderung wird vorteilhafterweise derart einstellbar ausgeführt, daß die Einspeiseleistung in der Meßstrecke größer ist als die gesteuerte Austragung durch die Vibrationsförderung. Hierdurch kann erreicht werden, daß die Meßstrecke stets voll von (durch die Vibrationsenergie) leicht gepreßten Meßgut ist.

Erst nach Beendigung des Materialflusses entleert sich die Meßstrecke durch Vibration vollständig und steht für die nächste Meßaufgabe bereit. Der Leer- und Vollstand der Meßstrecke kann über die gleiche Meßvorrichtung in der Anlagesteuerung verwendet werden, z. B. für Verriegelungsaufgaben. Die Vibrationsenergie verwendet man in vielen Fällen zur Vorbereitung von Schüttgutmustern für Labormessungen. Bei dem geschilderten besonders vorteilhaften Einsatz der Vibration wird durch diese eine erste Vorbereitungsstufe erzielt, der sich die Verdichtung dann als zweite Stufe anschließt.

Bei einer anderen vorteilhaften Ausgestaltung der Erfindung weist die Meßstrecke einen vorzugsweisen horizontalen Schneckenförderer auf, der die Zwangsförderung sicherstellt, wobei am Ende der Meßstrecke Rückstauelemente für das Meßgut angeordnet sind. Als Rückstauelemente können vorzugsweise eine unter dem Förderdruck sich öffnende Klappe oder fest eingebaute Drähte oder Lamellen benützt werden, wobei zweckmäßigerweise die Einrichtung zum zyklischen Verdichten des Meßgutes sowie der Meßaufnehmer im Bereich vor oder zwischen dem Schneckenförderer und/oder den Rückstauelementen anzuordnen sind.

Die Lösung mit Schneckenförderung bietet sich an in Fällen besonders schlecht fließfähiger Produkte oder solcher Produkte, die bei Vibration zum Zusammenbacken neigen, z. B. stark fetthaltige Produkte.

Die Erfindung betrifft auch ein Verfahren zur kontinuierlichen, quantitativen Bestimmung von Inhaltsstoffen in mehlförmigen oder anderen Nahrungsmittel-Mahlgütern, bei welchem das Mahlgut über eine Infrarot-Meßstrecke geleitet und dort verdichtet, das verdichtete Meßgut mit Infrarot-Licht bestrahlt und aus den vom Meßgut diffus zum Meßaufnehmer reflektierten Strahlen der Proteingehalt, Wassergehalt, Aschegehalt und/oder Farbwert der Meßprobe über eine Meßeinrichtung ermittelt wird.

Meßwerte in industriellen Anlagen haben nur dann einen "Sinn", wenn sie direkt im Zusammenhang mit dem zu vermessenden Produkt oder der vorzunehmenden Verarbeitung einsetzbar sind.

Die exakte Einhaltung des Protein- und Wassergehaltes zum Beispiel bei Mehl wird oftmals gesetzlich vorgeschrieben, muß aber in jedem Fall im Handel garantiert werden. Im Gegensatz zu einer Futtermühle, bei der das zu verarbeitende Produkt direkt und einmalig durch den Verarbeitungsprozeß geführt wird, muß bei einer Mehlmühle das Produkt teilweise einen wiederholten Verarbeitungsvorgang durchlaufen. Für die Herstellung von Mehl gibt es zwei grundsätzliche Wege, wobei je nach Besonderheit der Mühle auch Zwischenformen möglich sind:

1) Das Getreide wird durch entsprechende Führung direkt zu der vom Abnehmer gewünschten Mehlqualität verarbeitet, oder

2) es werden Mehlgrundtypen hergestellt, die später für die Qualitätsanforderungen des Kunden zusammengemischt werden.

In beiden Fällen ist aber eine bestmögliche Beherrschung der Verarbeitung/Reinigung /Mischung/Netzung/Vermahlung und Siebung usw. ein ganz wichtiges Produktionserfordernis. Damit wird die direkte Steuerbarkeit primärer Parameter mehr und mehr entscheidend. Auch hier ermöglicht die Erfindung einen entscheidenden weiteren Schritt im Hinblick auf die Regelung in der Mühle, indem Meßgut, also z. B. Mehl, nicht nur hohlraumfrei, sondern auch bewußt zusätzlich verdichtet und in diesem Zustand mit Infrarotlicht gemessen wird.

Das neue Verfahren ist dadurch gekennzeichnet, daß

- das Meßgut im Bereich des Meßaufnehmers zunächst leicht gepresst und geglättet wird,

- das leicht gepresste und geglättete Meßgut in senkrechter Richtung gegen den Meßaufnehmer zusätzlich verdichtet und stationär gehalten wird, und

- die aus der so verdichteten Meßprobe erhaltenen Meßsignale mittels erster Rechnermittel zu quantitativen Meßwerten verarbeitet werden.

Die Messungen können je nach Anwendungsfall beliebig oft wiederholt, vorzugsweise in einem vorgegebenen Zyklus durchgeführt werden, wobei vorzugsweise die Vorglättung und Vorpressung des Mahlgutes im Wege einer Zwangsförderung durchgeführt wird.

In vorteilhafter Weiterführung des Erfindungsgedankens werden durch Steuerung und Regelung der Mühle im Hinblick auf Protein- und/oder Wassergehalt des Mahlgutes die gewonnenen Meßwerte aufgrund von vorgegebenen Speicherwerten und zweiten Rechnermitteln direkt zur automatischen Steuerung der Rohmaterialmischung und/oder der Wasserzugabe und/oder der Mehlmischung eingesetzt. Dies kann über je einen oder mehrere echte Regelkreise für die nachfolgend angegebenen Faktoren erfolgen:

Protein - Rohmaterialmischung
Wassergehalt - Wasserzugabe
Protein - Mehlmischung.

Der Mühle können mittels eines übergeordneten Computers alle Werte (wie: Rohmaterialmischung, Wasserzugabe, Proteingehalt und Mehlqualität usw.) vorgegeben werden. Gleichzeitig kann der übergeordnete Computer Grenzwerte vorschreiben, innerhalb deren die erwähnten Regelkreise selbständig die Einstellungen der betreffenden Betriebseinrichtungen kontrollieren und nachführen.

Wird z. B. ein Regelkreis "Protein-Rohmaterialmischung" gebildet, werden (zumindest teilweise) die einzelnen Rohmaterialqualitäten gesondert bis zu der ersten Vermahlung behandelt, damit eine Korrektur direkt vor der Vermahlung (und daher mit dem geringstmöglichen Zeitverzug) erfolgen kann.

Sinngemäß soll bei der Wasserzugabe die Möglichkeit dieser Zugabe unmittelbar vor der Vermahlung gegeben sein; damit zumindest kleinere Korrekturen sofort wirksam sind (größere Änderungen der Wasserzugabe müssen allerdings in der Hauptvorbereitung vor der Abstehzelle vorgenommen werden). Selbstverständlich müssen bei der Regelung der Proteinwerte wie der Wasserwerte die Verzögerungen aus dem Mahlablauf stets berücksichtigt werden.

Bei der Anwendung des erfindungsgemäßen Verfahrens auf die Mehlzusammenstellung handelt es sich um einen Steuerungsvorgang, bei dem auf ein spezifisches Ziel hin (Erreichen eines Sollwertes) die notwendigen Steuerungs- und Umlenkbefehle zu erteilen sind. Das Gleiche gilt für die Glutenbeigabe zu Mehl.

Hinsichtlich der Werte für Mehlasche und Mehlfarbe kann sinngemäß vorgegangen werden, wenn auch, zumindest derzeit, einer Regelung der Mühle hinsichtlich Asche- und Farbwerten keine so große Bedeutung beigemessen wird.

Durch die Erfindung wird ein weiterer entscheidender Fortschritt für eine noch bessere Kontrolle und Steuerung der Mühle als ganzes möglich, insbesondere durch die Möglichkeit der Realisierung

einer direkten Regelung von Proteinwerten und

einer direkten Regelung von Wasserwerten.

Das erfindungsgemäße Verfahren läßt sich vorteilhaft dadurch weiterbilden, daß für die Durchführung einer Meßphase eine geringe Pressung des Mahlgutes durch Abstellen bzw. Stoppen der Zwangsförderung sichergestellt und durch mechanisches Verdichten des Meßgutes in dem Bereich des Meßaufnehmers die Meßprobe bereitgestellt sowie bis zur Messung aller Inhaltsstoffe (Protein, Wasser, Asche, Helligkeit) konstant unverändert) gehalten wird.

Aus Gründen der Betriebssicherheit ist es besonders günstig, wenn die Meßphasen in wählbaren Zeitabschnitten zyklisch wiederholt werden. Die Häufigkeit der Wiederholung richtet sich nach den besonderen Umständen des Verarbeitungsprozesses.

Ferner ist es besonders zweckmäßig, gegebenenfalls jede Einzelmessung während einer Meßphase zu wiederholen, die Meßergebnisse einer Gruppe von Meßphasen zu mitteln und als Ist-Werte den zweiten Rechnermitteln zur Steuerung bzw. Regelung der Rohmaterial-Mischung und/oder des Wassergehaltes und/oder der Mehlmischung zur Verfügung zu stellen, und die Produktparameter auf bestimmte vorgegebene Werte einzuregeln.

Die Erfindung ergibt als eine weitere Information nun auch ein Maß für die Gleichmäßigkeit des Mühlenlaufes, wobei aus dem Vergleich von mehreren Werten auf mögliche Störquellen geschlossen werden kann.

Zum Beispiel läßt sich eine plötzliche Zugabe von einigen Prozent Weizen mit erhöhtem Proteingehalt nach kurzem Zeitverzug ausdrucken, was auch eine rasche Erfassung z. B. solcher Änderungen durch die Bedienungsperson ermöglicht.

Die Erfindung umfaßt schließlich ein Verfahren zur Überwachung der Bestandteile (Inhaltsstoffe) von mehlartigen Nahrungsmitteln oder von Nahrungsmitteln-Mahlgütern, bei welchem

- das Mahlgut kontinuierlich über eine Infrarot-Meßstrecke geführt und dort verdichtet wird,

- das verdichtete Mahlgut laufend aufeinanderfolgend mit Infrarot-Licht bestrahlt und der Anteil der einzelnen Bestandteile durch Messung der Intensität des vom Mahlgut reflektierten Lichtes in den den einzelnen Bestandteilen zugeordneten Spektralbereichen ermittelt wird, wobei

- das Mahlgut für die laufend aufeinanderfolgenden Messungen zunächst in der Meßstrecke leicht gepreßt und auf seiner Oberfläche geglättet wird,

- anschließend zusätzlich senkrecht gegen den Meßaufnehmer verdichtet wird,

- während jeder Messung die Bewegung des Mahlgutesinnerhalb der Infrarot-Meßstrecke gestoppt wird,

- mittels eines ersten Rechners die Werte der Bestandteile ermittelt und jeweils mit einem von einem zweiten Rechner vorgegebenen Sollwert verglichen werden und

- in Abhängigkeit von den festgestellten Abweichungen zwischen ermittelten und vorgegebenen Werten Steuermaßnahmen (beispielsweise für Rohmaterialmischung, Mehlzusammenstellung, Wasserzugabe, Glutenbeigabe, Vorgabewerte für Mahlwalzeneinstellungen usw.) getroffen werden.

Nachfolgend wird nun die Erfindung anhand von Ausführungsbeispielen im Prinzip beispielshalber noch näher erläutert.

## Kurze Beschreibung der Zeichnungen

Es zeigen:

Figur 1 ein Prüfmuster entsprechend dem Stand der Technik;
Figur 2 das Prüfmuster aus Figur 1 nach geringer Pressung, z. B. in einer Förderschnecke;
Figur 3 schematisch ein Prüfmuster in verdichtetem Zustand gemäß der Erfindung;
Figur 4 das Verhältnis von Dichte (kg/cm³) über variierendem Druck P (kg/cm³) bei einem Mehlmuster;
Figur 5 eine schematische Schnittdarstellung einer erfindungsgemäßen Vorrichtung;
Figur 5a den Schnitt V - V in Figur 5;
Figur 6 eine andere Ausführungsform einer erfindungsgemäßen Vorrichtung mit einem Luftkissen als Einrichtung zum zyklischen Verdichten des Meßgutes;
Figur 6a den Schnitt VI - VI aus Figur 6;
Figur 7 eine weitere Ausführungsform einer erfindungsgemäßen Vorrichtung mit einem Löffel als Einrichtung zum zyklischen Verdichten des Meßgutes;
Figur 8 eine Prinzipdarstellung einer erfindungsgemäßen Anordnung mit einem Pneumatikkolben (wie bei Figur 5), wobei die Einheit über einem Schneckenförderer angeordnet ist:
Figur 9 eine Ansicht und Figur 10 einen Grundriß einer weiteren Ausführungsform der Erfindung, bei der die ganze Meßeinrichtung zwischen einer Förderschnecke und Rückstauelementen angeordnet ist;
Figur 11A schematisch eine erfindungsgemäße Steuerung der Rohmaterialmischung bei einer Mühle;
Figur 11B eine erfindungsgemäße Regelung der Wasserzugabe bei einer Mühle (schematisch);
Figur 12 schematisch eine Mischung von verschiedenen Mehlqualitäten nach der Erfindung.

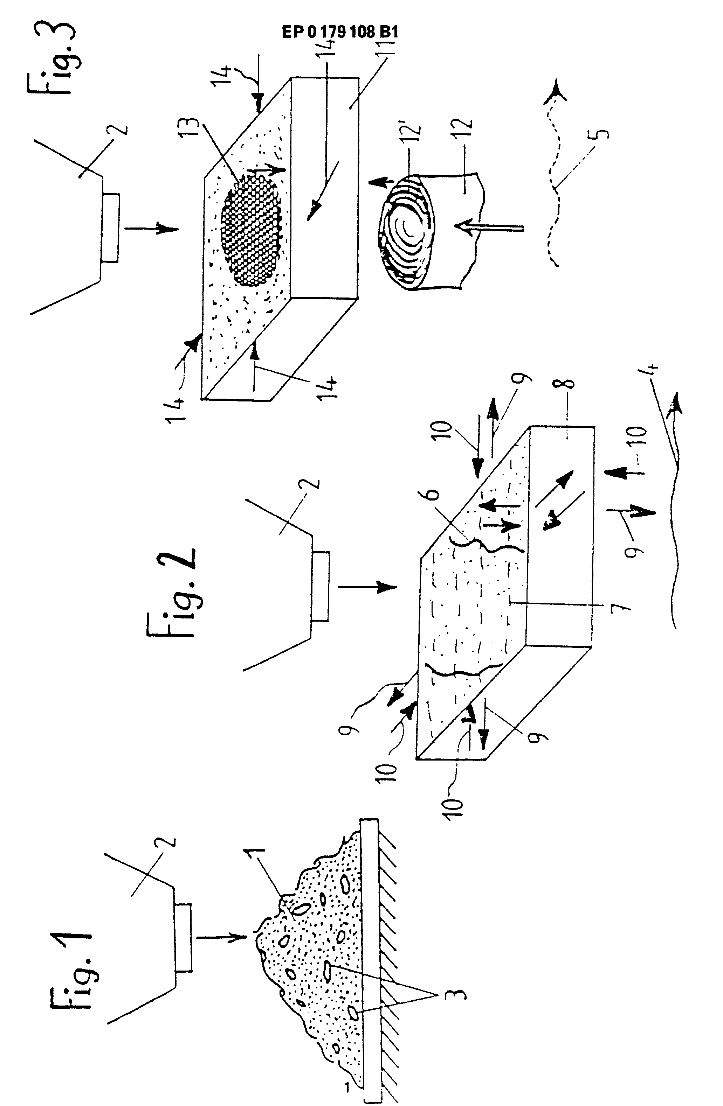

Die Figur 1 zeigt ein Häufchen 1 losen Mehles mit einer darauf gerichteten Infrarot-Meßoptik 2. Wie ersichtlich weist das Gut eine größere Anzahl von Gashohlräumen 3 auf, so daß eine Messung mittels Infrarot-Strahlen erfahrungsgemäß sehr ungenaue Resultate bringt. Sowohl die unregelmäßige Oberfläche wie die unkontrollierbaren Lufthohlräume verfälschen das Meßergebnis, da die Reflexion durch willkürliche Lage der Gutpartikel an der Oberfläche sowie des anders reagierenden Gases bzw. der Luft beeinflußt wird.

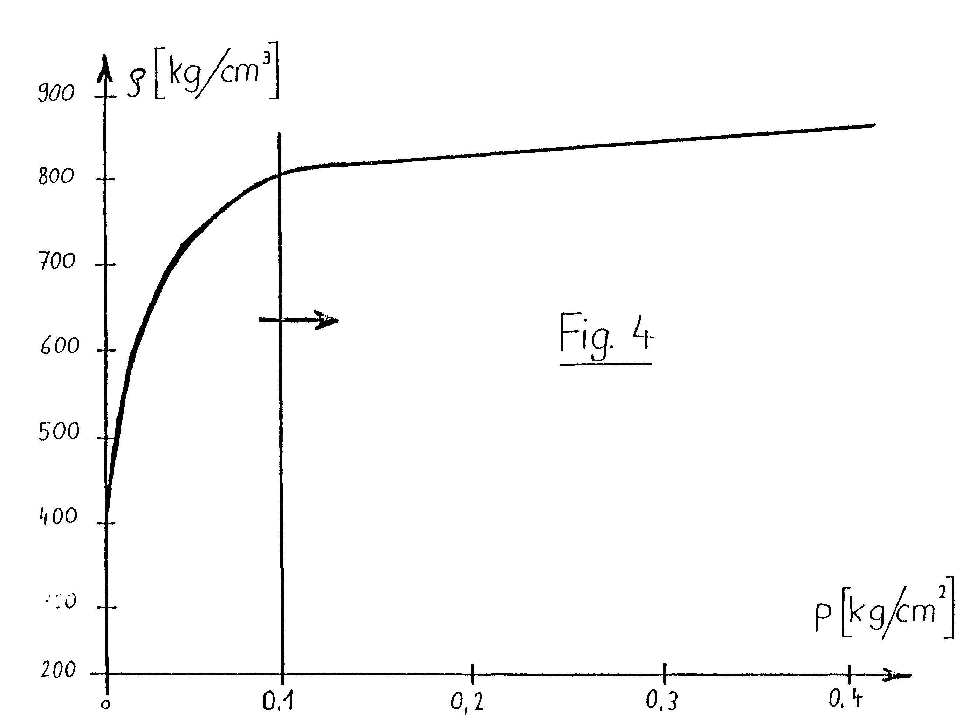

Die Erkenntnisse der Erfindung werden nun anhand der Figuren 2 und 3 dargestellt, wobei in beiden Figuren die Produktbewegung mit Pfeilen 4 bzw. 5 angegeben wird. Wird die Produktbewegung durch eine Zwangsförderung sichergestellt, dann führt dies zu einer geringen Pressung (z. B. bei Schneckenförderung). Die Förderung insbesondere im Falle eines Schneckenförderers kann unschwer so eingestellt werden, daß die Meßstrecke immer mit Produkt gefüllt ist. Vorzugsweise wird das Gehäuse des Schneckenförderes so lange gewählt, daß das schneckenfreie Ende ebensolang wie die Länge der Schnecke selbst ist, so daß ein Produktpfropfen mit der Schnecke gestoßen werden muß. Damit wird erreicht, daß das Produkt aus dem Meßbereich nicht entweichen und die Packungsdichte sich während der Messung nicht ändern kann. Durch Stoppen der Förderung wird dann sichergestellt, daß die Meßprobe unverändert bleibt.

Mehlige Güter weisen ein gutes Lufthaltevermögen auf. Beim Pressen von Mehl wird gleichzeitig auch die darin enthaltene Luft mitgepreßt. Stellt man die Schneckenförderung ab, wird nicht nur der Druck auf das Mehl weggenommen, sondern die im Mehl enthaltene Luft kann dann frei expandieren. Dadurch können sich feine Risse 6 ausbilden und wieder Höhlungen in der der Infrarot-Meßoptik 2 zugekehrte Mehloberflächeauftreten. Die Dichte des Produkts bleibt nur scheinbar gleich. Die Mehloberfläche, wie sie sich durch eine mechanische Zwangsförderung und eine mehr oder weniger rauhe Gehäuseinnenseite einstellt, führt je nach zufälliger Partikelzusammensetzung in Förderrichtung zu Rieffen 7, die sich störend auf Meßergebnisse auswirken können. Somit ist die Meßprobe 8 aufgrund der doch beachtlichen Kompressibilität von Mehl ohne zusätzlichen Eingriff in ihrer Beschaffenheit nicht konstant. Da sich die Ausdehnung im Mittenbereich des Pfropfens nicht auf beide Seiten erstreckt, das Produkt im Bereich der Infrarot-Meßoptik 2 also nahezu unbewegt ist, läßt sich dieser Sachverhalt nicht sogleich erkennen. Das Produktmuster dehnt sich nach allen Seiten aus (vgl. Pfeil 9) wodurch die vorherige Pressung (Pfeilrichtung 10) weitgehend wieder aufgehoben wird.

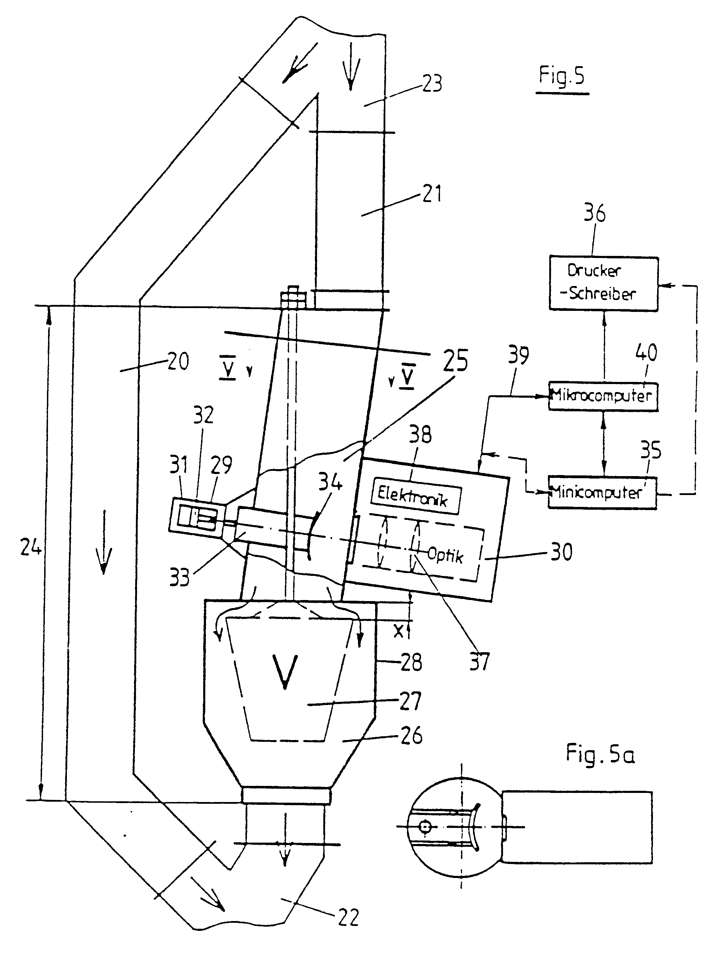

Mit der Erfindung werden nun aber diese möglichen Störquellen vollständig behoben. In der Meßstrecke 24 (Fig. 5), also in dem bereits vorgepreßten Gut, wird eine tatsächliche Verdichtung erzeugt, wie in Figur 3 dargestellt ist. Das Meßgut 11 wird durch einen Kolben 12 gegen die Meßoptik 2 gedruckt. Dabei wird nicht nur das Mehl verdichtet, sondern gleichzeitig werden vorhandene Lufteinschlüsse beseitigt, da Luft aus dem Bereich 13 hohen Druckes austritt. Der Druckbereich 13 ist schraffiert eingetragen, um anzudeuten, daß eine für den Zweck der Messung absolut gleichmäßige Oberfläche gebildet und so lange aufrechterhalten werden kann, bis der Meßvorgang abgeschlossen ist. Mit den Pfeilen 14 soll angedeutet werden, daß das Mehl vom Druckbereich 13 aus auf keine Seite entweichen kann, d.h. räumlich unter Druck gehalten wird. Der Bereich erhöhten Druckes verläuft kegelförmig und wird aufgrund der Schüttgutmechanik ausgebildet. Ist die Messung zu Ende, wird das ganze Meßgut 11 weggeschoben. Eine neue Messung kann zu beliebigem Zeitpunkt (bzw. zyklisch) wiederholt werden.

Die Figur 4 zeigt nun das Druck- und Kompressionsverhalten von Mehl. Erfindungsgemäß wird hier vorzugsweise im Bereich über 0,1 kg/cm$^2$, besonders vorzugsweise über 0,4 kg/cm$^2$ für den Verdichtungsdruck gearbeitet. Beste Resultate wurden im Bereich von 0,4 kg/cm$^2$ bis 1 kg/cm$^2$ ermittelt. Noch größere Drücke können zwar gewählt werden, haben aber den Nachteil, daß das Produkt teilweise verklumpt; bei sehr großen Drücken besteht sogar die Gefahr, daß das Mehl selbst beschädigt wird. Bei Versuchen wurde bei guten Ergebnissen mit einem Druck von 6 kg/cm$^2$ gearbeitet, wobei der Verdichtungskolben jedoch auf Anschlag lief.

Die Figuren 5 und 5a zeigen eine vollständige Meßstrecke 24 gemäß der Erfindung. An An eine Hauptleitung 20, in welcher der Hauptproduktstrom fließt, wird eine Abzweigleitung bzw. ein By-Pass 21 angeschlossen, der an seinem Ende über ein Rohrstück 22 wieder in die Hauptleitung 20 einmündet. Ein Überleitungsstück 23 stellt die Eingangsverbindung zwischen der Hauptleitung 20 und dem By-Pass 21 dar. Die Meßstrecke 24 weist einen oberen Preßrau 25 sowie einen Vibrauslauf 26 auf, in dem sich ein Vibrator 27 befindet, dessen Außenbegrenzung unten mit dem Gehäuse 28 des Vibroauslaufes 26 einen einstellbaren Dosierspalt X ausbilden. Dabei ist der Vibrator 27 im Gehäuse 28 pendelnd aufgehängt. Im Preßraum 25 sind in dessen unterem Drittel eine Einrichtung 29 zum zyklischen Verdichten des Mahlgutes sowie ein Meßaufnehmer 30 angeordnet. Die Druckmittel der Einrichtung 29 bestehen aus einem Pneumatikzylinder 31, einem pneumatischen Kolben 32 sowie einem Preßkolben 33, der bezüglich des Preßraumes längs der Achse des Pneumatikzylinders 31 verschiebbar ist. Der Preßkolben 33 weist seinerseits eine gewölbte Druckfläche 34 auf, die schaufelartig gegen den Meßaufnehmer 30 gerichtet ist. Im Meßaufnehmer 30 befindet sich eine Optik 37 sowie eine Auswertelektronik 38, von der digitale Signale über eine genormte Schnittstelle 39 an einen Mikrocomputer 40 bzw. einen Mikrocomputer 35 weitergeleitet werden. Der Mikrocomputer 40 kann direkt an einen Drucker 36 angeschlossen werden. Die Befehlseinheit für die ganze Meßstrecke 24 wird von dem Mikrocomputer 40 gebildet, der über die Auswertelektronik 38 den Vibrator 27 und den Pneumatikzylinder 31 steuert und gleichzeitig die Meßphase über den Meßaufnehmer 30 vorbereitet und einleitet.

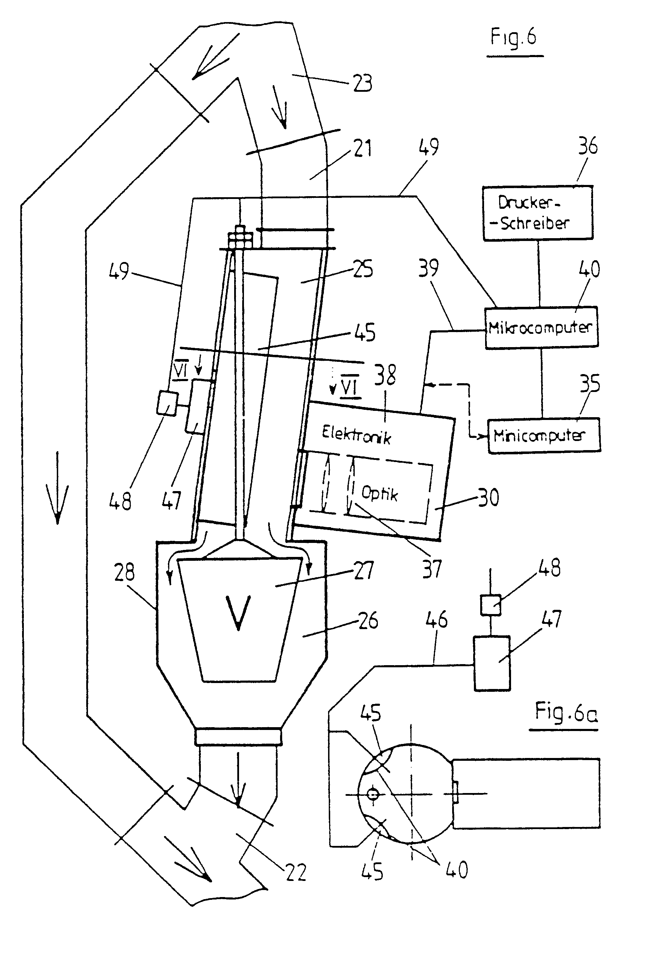

Bei der Darstellung der Figuren 6 und 6a besteht die Einrichtung zum Verdichten des Meßgutes aus zwei längs der Meßstrecke angeordneten, aufblasbaren Luftkissen 45, die innerhalb der Meßstrecke 25 angeordnet sind. Die beiden Luftkissen 45 werden über Pneumatikleitungen 46 sowie einen pneumatischen Druckgeber 47 gespeist und über einen Steuerkopf 48 gesteuert, der über ein elektrisches Steuerkabel 49 ebenso wie der Vibrator 27 von dem Mikrocomputer 40 angesteuert wird. Der Mikrocomputer 40 steuert auch hier das ganze Meßspiel, sinngemäß zu der Lösung gemäß Figur 5.

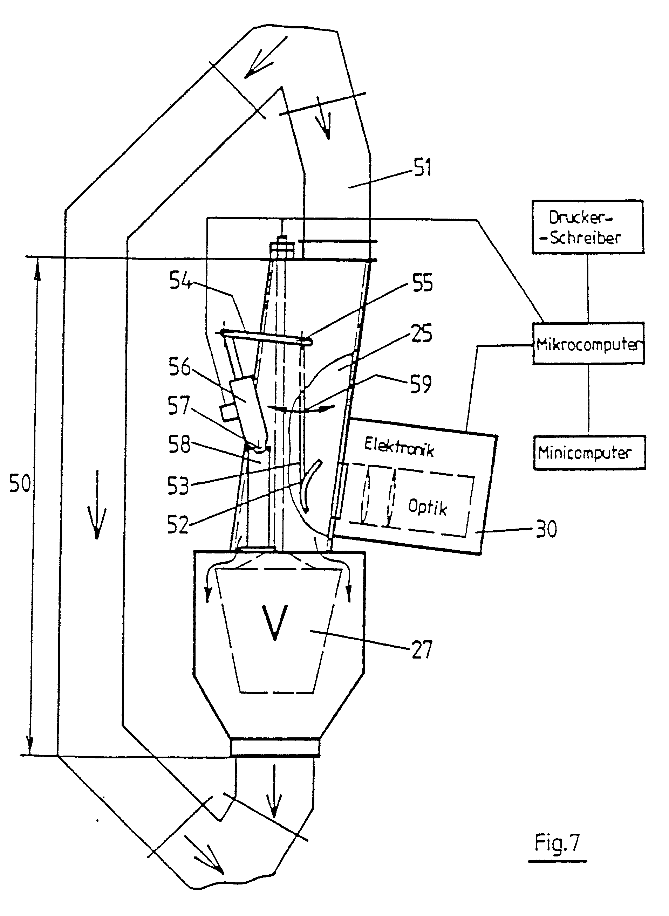

Der Grundaufbau der in Figur 7 gezeigten erfindungsgemäßen Vorrichtung entspricht im wesentlichen dem der Ausführungen aus den Figuren 5 und 6, allerdings weist er eine andere mechanische Einrichtung zum Verdichten des Meßgutes im Bereich des Meßaufnehmers 30 auf. Eine Meßstrecke 50 ist deshalb in Figur 7 etwas abgewandelt gegenüber der Darstellung nach den Figuren 5 und 6 gezeigt. In allen drei Ausführungsbeispielen der Figuren 5, 6 und 7 ist aber der Preßraum 25 als ein rohrähnliches Gehäuse ausgebildet. Ganz besonders wichtig ist es nun bei der Lösung gemäß den Figuren 5 und 7, daß sich der Preßraum 25 nach unten hin etwas erweitert. Die Querschnittsfläche der Meßstrecke, die hier vom Preßraum 25 gebildet wird, nimmt also nach unten hin zu. Damit kann nicht nur der Einfluß der Wandreibung auf das Produkt vermindert, sondern auch eine mögliche Störeinwirkung der mechanischen Einbauten im Preßraum 25 ausgeschaltet werden. In Figur 7 besteht die Einrichtung zum Verdichten des Meßgutes aus einem Löffel 52, der über einen Löffelhalter 53 innerhalb der Meßstrecke (d. h. des Preßraumes 25) um eine horizontale Achse 55 verschwenkbar ausgebildet ist. Der Löffelhalter 53 ist über die Achse 55 fest mit einem Hebel 54 verbunden und wird über einen Pneumatikzylinder 56 angetrieben, der für die erforderliche Verschwenkbewegung mittels eines Bolzens 57 gelenkig über eine Lagerstelle 58 an der Meßstrecke befestigt ist. Der Löffel 52 kann somit eine Bewegung (Pfeil 59) ausführen, die sinngemäß zu der Bewegung der gewölbten Druckfläche 34 in Figur 5 verläuft. Für die Messung wird der Löffel 52 mit dem beschriebenen Mechanismus gegen den Meßaufnehmer 30 bewegt, wodurch er das Meßgut vor dem Meßaufnehmer 30 verdichtet. Nach Beendigung einer Messung wird über die Steuerung der Löffel 52 von dem Meßaufnehmer 30 wieder entfernt, sodann der Vibrator 27 eingeschaltet und dadurch das verdichtete Gut wieder gelockert und nach unten ausgetragen.

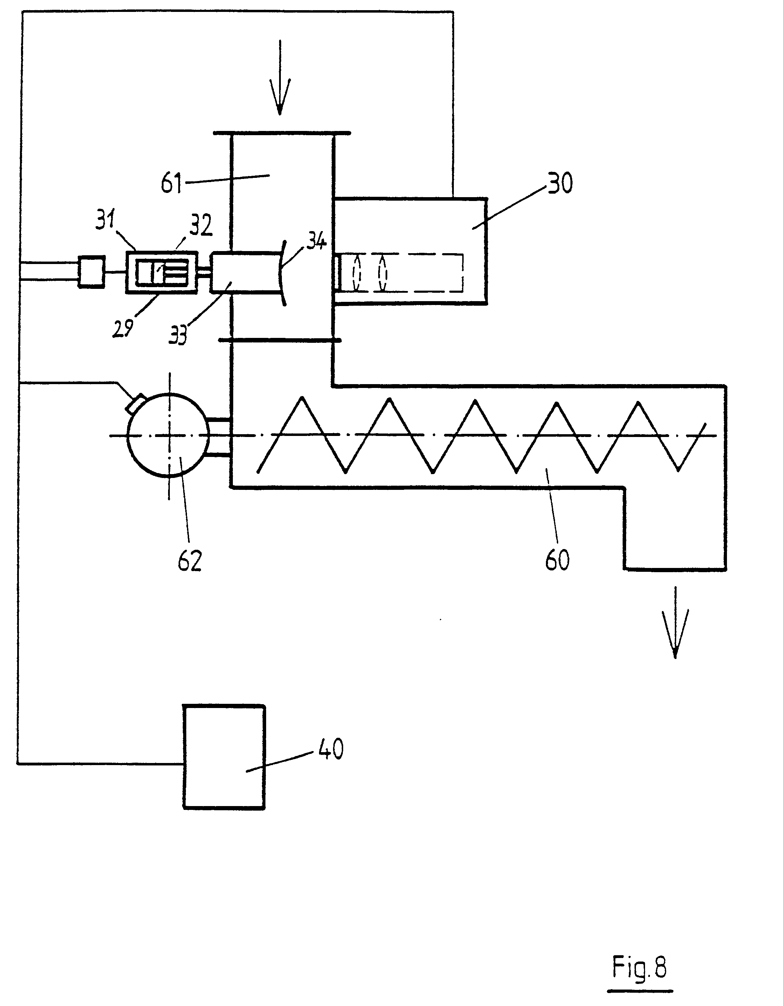

In Figur 8 ist der Einsatz des erfindungsgemäßen Meßverfahrens im Zusammenhang mit der Anwendung einer Dosierschnecke bzw. Förderschnecke 60 schematisch dargestellt. Der Meßaufnehmer 30 sowie die Einrichtungen zum Verdichten des Meßgutes 29, 31, 32, 33 und 34 sind sinngemäß zur Lösung nach der Figur 5 dargestellt. Anwendbar wäre hier aber auch die Lösung mit dem Löffel, wie in Figur 7 gezeigt. Die Förderschnecke 60 wird über (nicht dargestellte) Mittel derart angesteuert, daß ein Meßraum 61 während des Normalbetriebes stets mit entlüftetem Produkt gefüllt ist. Der Meßaufnehmer 30, die Einrichtung zum Verdichten des Produktes sowie ein Antriebsmotor 62 für die Förderschnecke 60 werden über den Mikrocomputer 40 angesteuert.

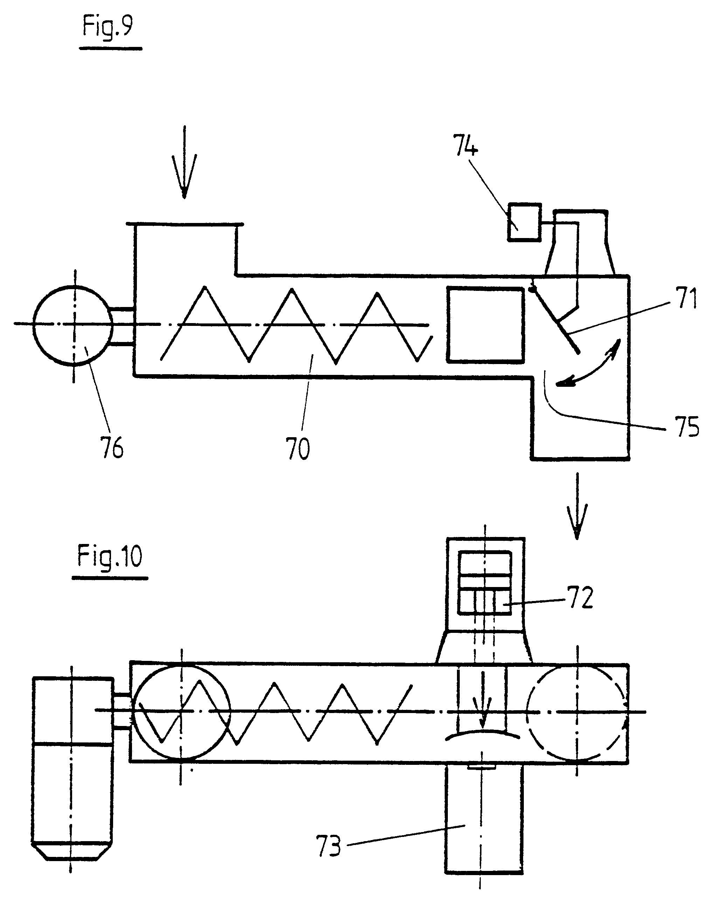

Die Figuren 9 und 10 zeigen eine zu Figur 8 abgeänderte Ausführungsform, wobei Figur 9 eine Prinzipdarstellung sowie Figur 8 und Figur 10 die Draufsicht auf die Vorrichtung nach Figur 9 zeigen. Eine Förderschnecke 70 weist hier im Bereich des Auslaufes ein Rückstauelement 71 auf, das über Antriebsmittel 74 im gewünschten Takt und der gewünschten Kraft gegen die Öffnung 75 bzw. von dieser weg bewegbar ist. Ein Antriebsmotor 76 und die Antriebsmittel 74 bzw. das Rückstauelement 71 werden so aufeinander abgestimmt, daß für die Messung ein bestimmter Druck im Meßgut aufrechterhalten wird. Die Förderschnecke 70 arbeitet gegen Druck. Für die Vorbereitung der Meßphase wird dieser Druck aufgebaut sowie eine Einrichtung 72 (Figur 10) zum Verdichten des Meßgutes betätigt und es werden sodann von einem Meßaufnehmer 73 die gewünschten Meßwerte aufgenommen. Nach Beendigung der Messung wird die Öffnung 75 wieder freigegeben und durch Einschalten des Antriebsmotors 76 der kontinuierliche Produktdurchsatz wieder in Gang gebracht.

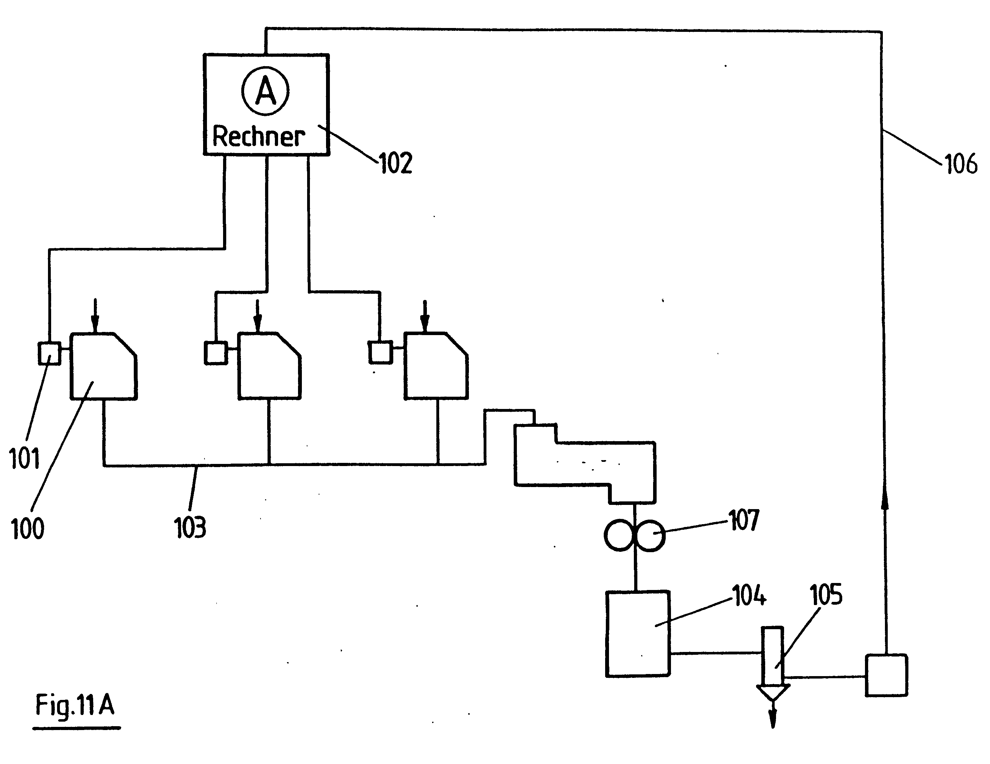

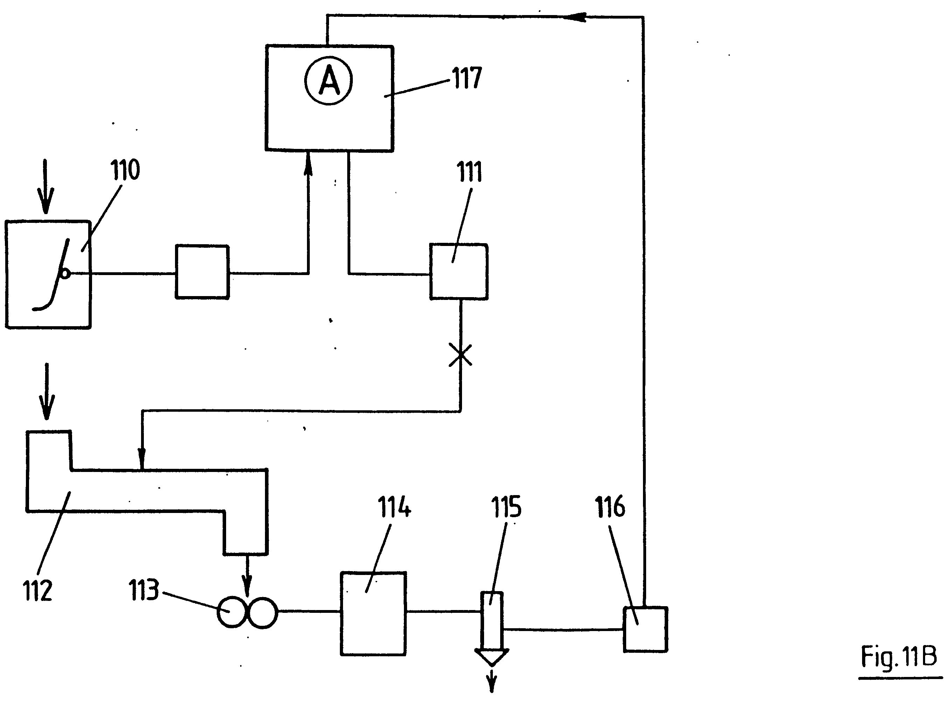

Figur 11A zeigt eine besonders vorteilhafte Anwendung des erfindungsgemäßen Meßverfahrens zum Steuern und Regeln der Produktmischung für die Herstellung von Mehl, Gries und Dunst in einer Mühle.

Für die jeweils eingelagerten Produktsorten werden über je ein kontinuierliches Durchflußmeßgerät 100 mit angebauter elektronischer Steuerung 101 über einen Rechner 102 die benötigten Mengenverhältnisse eingestellt. Die Produktmischung wird über eine gemeinsame Förderschnecke 103 in die Mühle bzw. auf die Walzenstühle 107 und Siebeinheiten 104 gegeben. Das gewonnene Mehl wird in einer Meßstrecke 105 bezüglich seiner Inhaltsstoffe, z. B. des Proteingehaltes, gemessen und die ermittelten Werte über Steuerleitungen 106 dem Rechner 102 eingegeben. Stellt nun der Rechner 102 Abweichungen von dem gewünschten (vorgegebenen) Protein-Sollwertes fest, korrigiert er automatisch die Mischung so lange (und unter Berücksichtigung der auftretenden Zeitverzögerung), bis der gewünschte Protein-Istwert mit dem Sollwert übereinstimmt.

In Figur 11B ist sinngemäß zu Figur 11A entsprechend die Regelung der Wasserzugabe gezeigt. Die Rohproduktmenge wird über einen automatischen Mengenregler 110 kontinuierlich gemessen und entsprechend einem gewünschten Sollwert die hierfür erforderliche Wassermenge automatisch über einen Wasserdosierer 111 zudosiert. Rohmaterial und Wasser werden in einem Intensivnetzer 112 vermischt, in Walzenstühlen 113 vermahlen, in Plansichtern 114 das Mehl ausgesiebt und die Inhaltsstoffe, hier der Wassergehalt des Mehles, in einer Meßstrecke 115 gemessen. Das Meßsignal des Infrarotmeßgerätes 116 wird über eine Steuerleitung an einen Rechner 117 gegeben, der den Istwert/Sollwert-Vergleich vornimmt und Abweichungen gemäß einem eingespeicherten Progamm durch entsprechende Änderung der Wasserzugabe über den Wasserdosierer 111 korrigiert.

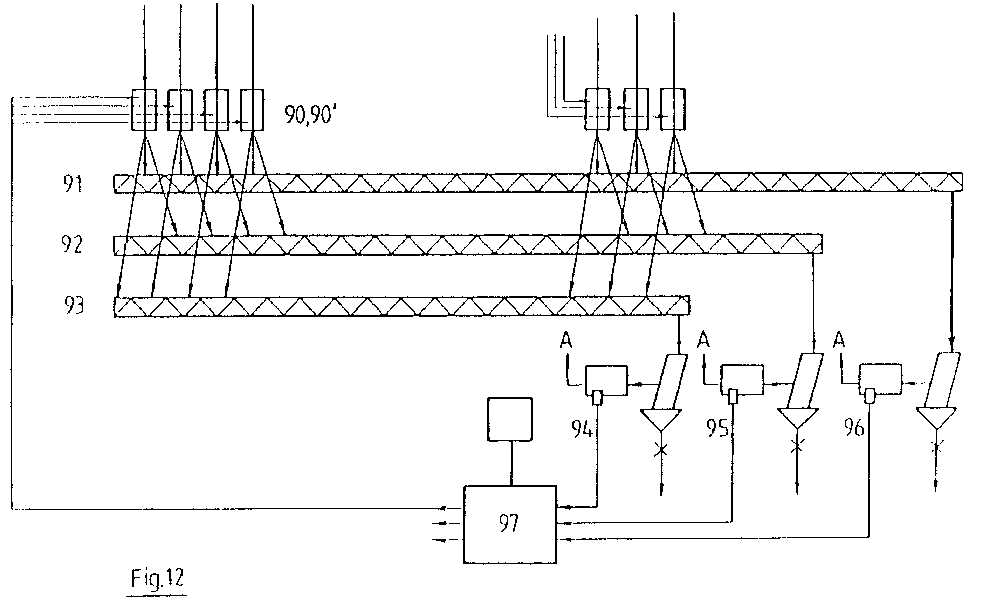

Figur 12 zeigt eine weitere interessante Anwendung des neuen Verfahrens für die Herstellung einer Mehlmischung unter Vorgabe bestimmter gewünschter Werte der enthaltenen Inhaltsstoffe, zum Beispiel Protein, Asche und Farbe. Die Stutzen 90 stellen die Ausläufe von Sichteranteilen dar, die auf eine von drei Mischschnecken 91, 92, 93 führbar sind.

An den Stutzen 90 ist jeweils eine Rohrweiche 90' angebracht, die aufgrund der Abweichungen zwischen gewünschtem Sollwert und Istwert (Meßwert) der jeweils gewonnenen Inhaltsstoffe von einem Rechner 97 angesteuert werden. An jedem Ausgang der Mischschnecken 91, 92 und 93 ist je eine Meßstrecke 94, 95 und 96 für die Ermittlung der Meßwerte vorgesehen.

Das erfindungsgemäße Verfahren ermöglicht erstmals eine Regelung auch einzelner Parameter bei der Walzenmahlung, zum Beispiel des Walzendruckes, indem z. B. eine Proteinbeschädigung durch Verminderung des Mahldruckes automatisch überwacht werden kann und entsprechende Steuerbefehle auslösbar sind.

Die Einrichtung zur Verdichtung des Meßgutes kann vorteilhafterweise auch zum Beispiel im Bereich der Druckfläche 34 (Figur 5) ein federndes oder elastisches Element zum Konstanthalten des Verdichtungsdruckes aufweisen. Sinngemäß hierzu könnte der Löffelhalter 53 als federndes Element ausgebildet werden. Hierdurch kann der pneumatische Kolben "auf Anschlag" gefahren und ein Restdruck durch die verbleibende Federspannkraft aufrechterhalten werden. Damit aber läßt sich ein geringes Nachgeben des verdichteten Produkts durch ein entsprechendes Nachlaufen der Einrichtung zum Verdichten des Meßgutes kompensieren.

**Patentansprüche**

1. Infrarot-Meßeinrichtung für die kontinuierliche, quantitative Bestimmung einzelner Bestandteile von Mehl oder anderen Nahrungsmittel-Mahlgütern, in welcher
a) das Mahlgut (11) durch eine rohrförmige Meßstrecke (24, 25, 50, 105, 115) geführt und dort im Bereich eines Meßaufnehmers (2, 30, 73) verdichtet wird gekennzeichnet, durch
b) Mittel (27, 60, 70) zum leichten Pressen und Glätten des Mahlgutes (11) in der Meßstrecke (24, 25, 50, 105, 115),
c) eine Einrichtung (12, 29, 31, 32, 33, 34, 45, 52, 72) zum zusätzlichen Verdichten des vorgepreßten Meßgutes (11) in senkrechter Richtung gegen den Meßaufnehmer (2, 30, 73) und
d) Mittel (35, 40) zum Stoppen der Bewegung des so verdichteten Meßgutes (11) und Auslösen der eigentlichen Infrarot-Meßphase.

2. Meßvorrichtung nach Anspruch 1, dadurch gekennzeichnet, daß die Mittel zum leichten Pressen und Glätten des Meßgutes (11) Zwangsfördermittel (27, 60, 70) sind.

3. Meßvorrichtung nach Anspruch 1 oder 2 dadurch gekennzeichnet, daß eine Steuereinrichtung (40) vorgesehen mittels derer die Zwangsförderung (27, 60, 70) während der Dauer einer Infrarotmeßphase unterbrechbar ist.

4. Meßvorrichtung nach einem der vorstehenden Ansprüche, dadurch gekennzeichnet, daß eine daß unabhängige Steuereinrichtungen (35, 40) für die Unterbrechung der Zwangsförderung, für die zyklische Verdichtung des Meßgutes und für das Auslösen sowie Steuern der Infrarotmeßphase vorgesehen sind.

5. Meßvorrichtung nach einem der vorstehenden Ansprüche dadurch gekennzeichnet, daß die Meßstrecke (24, 25, 50, 105, 115) im Bereich des Meßaufnehmers (30, 73) in Form eines Meßrohres ausgebildet ist.

6. Meßvorrichtung nach Anspruch 5, dadurch gekennzeichnet, daß die optische Achse des Meßaufnehmers (30, 73) im wesentlichen senkrecht zur Meßgutoberfläche ausgerichtet ist.

7. Meßvorrichtung nach einem der vorstehenden Ansprüche dadurch gekennzeichnet, daß die Einrichtung (29, 31, 32, 33, 34, 45, 52, 72) zum zyklischen Verdichten des Meßgutes einen Druckkörper (32, 33, 34, 45, 52, 53, 72) aufweist, der in Richtung auf den Meßaufnehmer (30, 73) hin verschiebbar ist.

8. Meßvorrichtung nach einem der vorstehenden Ansprüche dadurch gekennzeichnet, daß dem Druckkörper (34, 52) ein weg- und druckeinstellbarer Pneumatikzylinder (31, 56) zugeordnet ist.

9. Meßvorrichtung nach einem der Ansprüche 1 bis 7, dadurch gekennzeichnet, daß der Druckkörper (34, 52) Teil eine weg- und krafteinstellbaren Magnetspule ist.

10. Meßvorrichtung nach einem der Ansprüche 1 bis 7, dadurch gekennzeichnet, daß der Druckkörper (52, 53) als bewegbarer Löffel (52) ausgebildet

11. Meßvorrichtung nach Anspruch 10, dadurch gekennzeichnet, daß der Löffel (52) verschwenkbar und über pneumatische oder elektrische Antriebsmittel gegen den Meßaufnehmer (30) hin im wesentlichen in Richtung der optischen Achse bewegbar ist.

12. Meßvorrichtung nach Anspruch 11, dadurch gekennzeichnet, daß der Löffel (52) in einer einem Suppenlöffel ähnlichen Form ausgebildet ist, wobei seine konkave Seite, d. h. Löffelinnenseite dem Meßaufnehmer (30) zugewendet und dadurch das Meßgut mittels des Löffels (52) gegen den Meßaufnehmer (30) hin verdichtbar ist.

13. Meßvorrichtung nach einem der Ansprüche 1 bis 7, dadurch gekennzeichnet, daß die Einrichtung zum zyklischen Verdichten des Meßgutes aufblasbare Druckkissen (45) aufweist, die an der Meßstrecke (24) dem Meßaufnehmer (30) gegenüberliegend angeordnet sind.

14. Meßvorrichtung nach Anspruch 13, dadurch gekennzeichnet, daß das Druckkissen (45) längs der Meßstrecke (24) angeordnet ist und sich über eine Länge erstreckt, die ein Mehrfaches der Längsabmessung des Meßaufnehmers beträgt.

15. Meßvorrichtung nach einem der Ansprüche 1 bis 7, dadurch gekennzeichnet, daß die Einrichtung zum zyklischen Verdichten des Meßgutes als ein elektrisch oder pneumatisch betätigbarer Kolben (32) ausgeführt ist.

16. Meßvorrichtung nach einem der Ansprüche 1 bis 15, dadurch gekennzeichnet, daß die Meßstrecke (24) als eine Vibromeßstrecke ausgebildet ist, wobei die Zwangsförderung durch einen Vibrator (27) erfolgt.

17. Meßvorrichtung nach Anspruch 16, dadurch gekennzeichnet, daß die Vibromeßstrecke als By-Pass (21) einer Hauptleitung (20) ausgebildet ist.

18. Meßvorrichtung nach Anspruch 16 oder 17, dadurch gekennzeichnet, daß der Vibrator (27) eine glockenartige Form aufweist und im inneren der Meßstrecke pendelnd aufgehängt ist, wobei er für die Meßstrecke (24) einerseits durch die Vibration als Zwangsfördermittel dient und andererseits durch die Glockenform und den entsprechenden Wandteil eines Gehäuses (28) am unteren Ende der Meßstrecke (24) einen Dosierspalt ("X") bildet.

19. Meßvorrichtung nach einem der Ansprüche 16 bis 18, dadurch gekennzeichnet, daß die Meßstrecke (24) im Überlaufprinzip angeordnet und in einem By-Pass (21) angeordnet ist.

20. Meßvorrichtung nach einem der Ansprüche 16 bis 19, dadurch gekennzeichnet, daß die Dosierleistung der Vibrationsförderung derart einstellbar ist, daß die Einspeiseleistung in die Meßstrecke (24) größer als die gesteuerte Austragung durch die Vibrationsförderung ist, wodurch die Meßstrecke (24) stets voll von durch die Vibration leicht gepreßtem Mahlgut ist.

21. Meßvorrichtung nach einem der Ansprüche 1 bis 15, dadurch gekennzeichnet, daß die Meßstrecke (24) einen vorzugsweise horizontal angeordneten Schneckenförderer (70) für die Zwangsförderung aufweist und am Ende der Meßstrecke (24) ein Rückstau-Element (71) für das Meßgut angeordnet ist.

22. Meßvorrichtung nach Anspruch 21, dadurch gekennzeichnet, daß das Rückstau-Element (71) eine unter dem Förderdruck sich öffnende Klappe (71) oder fest eingebaute Drähte oder Lamellen aufweist.

23. Meßvorrichtung nach Anspruch 21 oder 22, dadurch gekennzeichnet, daß die Einrichtung (29, 31, 32, 33, 34, 45, 52, 72) zum zyklischen Verdichten des Meßgutes sowie der Meßaufnehmer (30, 73) im Bereich vor oder zwischen dem Schneckenförderer (60, 70) und/oder dem Rückstau-Element (71) angeordnet sind.

24. Verfahren zur kontinuierlichen, quantitativen Bestimmung von Inhaltsstoffen in mehlförmigen oder anderen Nahrungsmittel-Mahlgütern, bei welchem

a) das Mahlgut (11) über eine Infrarot-Meßstrecke (24, 25, 50, 105, 115) geleitet und dort verdichtet wird,

b) das verdichtete Mahlgut (11) mit Infrarot-Licht bestrahlt und aus den von der Meßprobe diffus zum Meßaufnehmer (2, 30, 73) reflektierten Strahlen der Proteingehalt, Wassergehalt, Aschegehalt und/oder Farbwert der Meßprobe über eine Meßeinrichtung (29, 31, 32, 33, 34, 45, 52, 72) ermittelt wird,

dadurch gekennzeichnet, daß

c) das Mahlgut (11) im Bereich des Meßaufnehmers (2, 30, 73) zunächst leicht gepreßt und leicht geglättet wird,

d) das leicht gepreßte und geglättete Mahlgut (11) in senkrechter Richtung gegen den Meßaufnehmer (2, 30, 73) zusätzlich verdichtet und stationär gehalten wird, und

e) die aus der so verdichteten Meßprobe erhaltnen Meßsignale mittels erster Rechnermittel zu quantitativen Meßwerten verarbeitet werden.

25. Verfahren nach Anspruch 24, dadurch gekennzeichnet, daß die leichte Pressung und Glättung des Mahlgutes (11) im Wege einer Zwangsförderung durchgeführt wird.

26. Verfahren nach Anspruch 24 oder 25, dadurch gekennzeichnet, daß durch Steuerung und Regelung der Mühle im Hinblick auf den Protein- und/oder Wassergehalt des Mahlgutes die gewonnenen Meßwerte aufgrund von vorgegebenen Speicherwerten und zweiten Rechnermitteln direkt zur automatischen Steuerung der Rohmaterialmischung und/oder der Wasserzugabe und/oder Mehlmischung eingesetzt werden.

27. Verfahren nach einem der Ansprüche 24 bis 26, dadurch gekennzeichnet, daß für die Durchführung einer Meßphase eine leichte Pressung bzw. Hohlraumfreihaltung des Mahlgutes durch Abstellen bzw. Stoppen der Zwangsförderung sichergestellt und durch mechanisches Verdichten des Meßgutes in dem Bereich des Meßaufnehmers die Meßprobe bereitgestellt sowie die verdichtete Meßprobe zur Messung der Inhaltsstoffe, z. B. Protein, Wasser, Asche, Helligkeit konstant gehalten wird.

28. Verfahren nach einem der Ansprüche 24 bis 26, dadurch gekennzeichnet, daß die Meßphasen in wählbaren Zeitabschnitten zyklisch wiederholt werden.

29. Verfahren nach einem der Ansprüche 24 bis 26, dadurch gekennzeichnet, daß gegebenenfalls jede Einzelmessung während einer Meßphase wiederholt, die Meßergebnisse einer Gruppe Meßphasen gemittelt und als Ist-Werte den zweiten Rechnermitteln zur Steuerung der Rohmaterialmischung und/oder des Wassergehaltes und/oder der Mehlmischuhg zur Verfügung gestellt und die Produktparameter auf bestimmte vorgegebene Protein und Wasserwerte geregelt werden.

30. Verfahren nach Anspruch 24, dadurch gekennzeichnet, daß bei der Ausführung jeweils einer Meßphase die Verdichtung des dabei unbewegten Mahlgutes während der Dauer der Messung im wesentlichen konstant gehalten wird.

31. Verfahren nach einem der Ansprüche 24 bis 30, dadurch gekennzeichnet, daß zum mechanischen Verdichten der Meßprobe ein elastisches oder federndes Element zusätzlich eingesetzt wird.

32. Verfahren nach einem der Ansprüche 24 bis 31 dadurch gekennzeichnet, daß während einer Meßphase mehrere Messungen für jeden einzelnen Spektralbereich vorgenommen und aus den mehreren Meßergebnissen ein Mittelwert gebildet wird, der dann mit dem Vorgabewert für den betreffenden Spektralbereich verglichen wird.

33. Verfahren nach einem der Ansprüche 24 bis 32 dadurch gekennzeichnet, daß die Meßergebnisse aus einer Anzahl einzelner Meßphasen gemittelt und der so errechnete Mittelwert als Ist-Wert an einen weiteren Rechner angelegt wird.

34. Verfahren zur Überwachung der Bestandteile, d. h. Inhaltsstoffe von mehlartigen Nahrungsmitteln oder von Nahrungsmittel-Mahlgütern, bei welchem

a) das Mahlgut kontinuierlich über eine Infrarot-Meßstrekke (24, 25, 50, 105, 115) geführt und dort verdichtet wird,

b) das verdichtete Mahlgut laufend aufeinanderfolgend mit Infrarot-Licht bestrahlt und der Anteil der einzelnen Bestandteile durch Messung der Intensivität des vom Mahlgut reflektierten Lichtes in den den einzelnen Bestandteilen zugeordneten Spektralbereichen ermittelt wird,

dadurch gekennzeichnet, daß

c) das Mahlgut für die laufend aufeinanderfolgenden Messungen zunächst in der Meßstrecke (24, 25, 50, 105, 115) leicht gepreßt und auf seiner Oberfläche geglättet wird,

d) anschließend das vorgepreßte Mahlgut zusätzlich senkrecht gegen den Meßaufnehmer (2, 30, 73) verdichtet wird,

e) während jeder Messung die Bewegung der Meßprobe (11) innerhalb der Infrarot-Meßstrecke (24, 25, 50, 105, 115) gestoppt wird,

f) mittels eines ersten Rechners die Anteilwerte der Bestandteile ermittelt und jeweils mit von einem zweiten Rechner vorgegebenen Sollwerten verglichen werden und

g) in Abhängigkeit von den festgestellten Abweichungen zwischen ermittelten und vorgegebenen Werten Steuermaßnahmen, beispielsweise für Rohmaterialmischung, Mehlzusammenstellung, Waßerzugabe, Glutenbeigabe, Vorgabewerte für Mahlwalzeneinstellungen, getroffen werden.

**Claims**

1. An infrared measuring device for the continuous quantitative determination of individual constituents of flour or other ground food stocks wherein

a) the ground stock (11) is passed through a tubular test section (24, 25, 50, 105, 115) and is there compacted in the range of a measuring means (2, 30, 73)

characterised by

b) means (27, 60, 70) for the light compression and smoothing of the ground stock (11) in the test section (24, 25, 50, 105, 115),

c) a device (12, 29, 31, 32, 33, 34, 45, 52, 72) for the additional compacting of the precompressed test stock (11) in the vertical direction against the measuring means (2, 30, 73) and

d) means (35, 40) for stopping the movement of the test stock (11) compacted in this way and starting the actual infrared measuring stage.

2. A measuring device according to claim 1, characterised in that the means for the light compression and smoothing of the test stock (11) are constrained conveyance means (27, 60, 70).

3. A measuring device according to claim 1 or 2, characterised in that provision is made for a control device (40), whereby the constrained conveyance (27, 60, 70) can be interrupted during the period of an infrared measuring stage.

4. A measuring device according to one of the preceding claims, characterised in that provision is made for independent control devices (35, 40) for the interruption of the constrained conveyance, for the cyclic compacting of the test stock and for the starting, as well as the control of the infrared measuring stage.

5. A measuring device according to one of the preceding claims, characterised in that in the range of the measuring means (30, 73), the test section (24, 25, 50, 105, 115) is designed in the form of a measuring tube.

6. A measuring device according to claim 5, characterised in that the optical axis of the measuring means (30, 73) is orientated substantially vertically to the surface of the test stock.

7. A measuring device according to one of the preceding claims, characterised in that the device (29, 31, 32, 33, 34, 45, 52, 72) for the cyclic compacting of the test stock comprises a pressure means (32, 33, 34, 45, 52, 53, 72) which is displaceable towards the measuring means (30, 73).

8. A measuring device according to one of the preceding claims, characterised in that a pneumatic cylinder (31, 56) whose travel and pressure can be adjusted, is assigned to the pressure means (34, 52).

9. A measuring device according to one of claims 1 to 7, characterised in that the pressure means (34, 52) forms part of a magnetic coil whose displacement and power can be adjusted.

10. A measuring device according to one of claims 1 to 7, characterised in that the pressure means (52, 53) is designed as a movable spoon (52).

11. A measuring device according to claim 10, characterised in that the spoon (52) is tiltable and can be moved by means of pneumatic or electric drive means towards the measuring means (30), substantially in the direction of the optical axis.

12. A measuring device according to claim 11, characterised in that the spoon (52) is designed in a form similar to a soup spoon, its concave side, that is to say, the inner side of the spoon, being turned towards the measuring means (30) and the test stock thereby being capable of being compacted by means of the spoon (52) against the measuring means (30).

13. A measuring device according to one of claims 1 to 7, characterised in that the device for the cyclic compacting of the test stock comprises inflatable pressure pads (45) arranged on the test section (24) opposite the measuring means (30).

14. A measuring device according to claim 13, characterised in that the pressure pad (45) is arranged along the test section (24) and extends over a length amounting to a multiple of the longitudinal dimension of the measuring means.

15. A measuring device according to one of claims 1 to 7, characterised in that the device for the cyclic compacting of the test stock is designed as an electrically or pneumatically actuable piston (32).

16. A measuring device according to one of claims 1 to 15, characterised in that the test section (24) is designed as a vibrating test section, the constrained conveyance being effected by a vibrator (27).

17. A measuring device according to claim 16, characterised in that the vibrating test section is designed as a by-pass (21) of a main line (20).

18. A measuring device according to claim 16 or 17, characterised in that the vibrator (27) is bell-shaped and is suspended for oscillation inside the test section, in which arrangement it serves on the one hand, as the constrained conveyance means for the test section (24) because of the vibration and it forms on the other hand, because of the bell shape and the corresponding wall portion of a casing (28), a dosing gap ("X") at the lower end of the test section (24).

19. A measuring device according to claim 16 to 18, characterised in that the test section (24) is arranged according to an overflow principle and is arranged in a bypass (21).

20. A measuring device according to one of claims 16 to 19, characterised in that the dosing output of the vibration conveyance can be adjusted in such a way that the infeed into the test section (24) is greater than the controlled discharge through the vibration conveyance, as a result of which the test section (24) is always filled with ground stock slightly compressed by the vibration.

21. A measuring device according to one of claims 1 to 15, characterised in that the test section (24) comprises a screw conveyor (70) for the constrained conveyance, preferably arranged horizontally, and that a reflux element (71) for the test stock is arranged at the end of the test section (24).

22. A measuring device according to claim 21, characterised in that the reflux element (71) has a flap (71) opening under the conveyance pressure or fixedly installed wires or strips.

23. A measuring device according to claim 21 or 22, characterised in that the device (29, 31, 32, 33, 34, 45, 52, 72) for the cyclic compacting of the test stock, as well as the measuring means (30, 73) are arranged in the zone ahead of, or between the screw conveyor (60, 70) and/or the reflux element (71).

24. A method for the continuous quantitative determination of constituents in flour-type or other ground food stocks wherein

a) the ground stock (11) is passed through an infrared test section (24, 25, 50, 105, 115) and is there compacted,

b) the compacted ground stock (11) is irradiated with infrared light and the protein content, water content, ash content and/or colour value of the test sample is determined by a measuring device (29, 31, 32, 33, 34, 45, 52, 72) from the diffuse rays reflected from the test sample to the measuring means (2, 30, 73)

characterised in that

c) the ground stock (11) is first lightly compressed and lightly smoothed in the range of the measuring means (2, 30, 73)

d) the lightly compressed and smoothed ground stock (11) is additionally compacted in the vertical direction against the measuring means (2, 30, 73) and held stationary and

e) and the test signals obtained from the test sample compacted in this way are processed by first computing means into quantitative measurement data.

25. A method according to claim 24, characterised in that the light compression and smoothing of the ground stock (11) is effected by way of a constrained conveyance.

26. A method according to claim 24 or 25, characterised in that by the control and regulation of the mill with a view to the protein and/or water content of the ground stock, the measured data, obtained on the basis of predetermined stored values and second computing means, are directly used for the automatic control of the raw materials mix and/or the addition of water and/or the flour mix.

27. A method according to one of claims 24 to 26, characterised in that for carrying out the test stage, a light compression or maintenance of the cavities of the ground stock is secured by the switching off or stopping of the constrained conveyance and that the test sample is prepared by mechanically compacting the ground stock in the range of the measuring means and that the compacted test sample is kept constant for measuring the constituents e. g. protein, water, ash, whiteness.

28. A method according to one of claims 24 to 26, characterised in that the measuring stages are repeated in cycles at selectable intervals.

29. A method according to one of claims 24 to 26, characterised in that if required, each individual measurement is repeated during one measuring stage, that the test results of a group of measuring stages is averaged and is placed at the disposal of the second computing means as the actual values for controlling the raw materials mix and/or water content and/or the flour mix and that the product parameters are adjusted to fixed predetermined protein and water values.

30. A method according to claim 24, characterised in that in the execution of a respective measuring stage, the compacting of the ground stock stationary therein, is kept substantially constant during the period of the measurement.

31. A method according to one of claims 24 to 30, characterised in that for the mechanical compacting of the test sample, an elastic or resilient element is additionally used.

32. A method according to one of claims 24 to 31, characterised in that during one measuring stage, several measurements are effected for each individual spectral range and that a mean value is formed from the several measurements which is then compared with the reference value for the spectral range concerned.

33. A method according to one of claims 24 to 32, characterised in that the test results from a number of individual measuring stages are averaged and that the mean value obtained in this way is applied to a further computer as the actual value.

34. A method for the monitoring of the constituents i.e. ingredients of flour type foods or of ground food stocks wherein

a) the ground stock is continuously passed through an infrared test section (24, 25, 50, 105, 115) and is there compacted,

b) the compacted ground stock is regularly [and] successively irradiated with infrared light and the proportion of the individual constituents is determined by the measurement of the light reflected by the ground stock in the spectral ranges associated with the individual constituents,

characterised in that

c) for the regular consecutive measurements, the ground stock is first lightly compressed and smoothed on its surface in the test section (24, 25, 50, 105, 115),

d) the precompressed ground stock is additionally compacted against the measuring means (2, 30, 73).

e) during each measurement, the movement of the test sample (11) is stopped within the infrared measuring test section (24, 25, 50, 105, 115),

f) the proportions of the constituents are determined by means of a first computer and are respectively compared with the preset required values supplied by a second computer and

g) controlling measures are taken in accordance with the established differences between the ascertained and preset values, for example, for the raw materials the flour composition, the addition of water, addition of gluten, [and] for setting the values for the adjustment of the grinding rollers.

**Revendications**

1. Installation de mesure aux infrarouges pour l'analyse quantitative continue de différents constituants d'une farine ou d'autres matières moulues pour produits alimentaires, dans laquelle:

a) la matière moulue (11) est dirigée a l'aide d'une voie tubulaire de mesure (24, 25, 50, 105, 115) et y est tassée dans la zone d'un capteur de mesure (2, 30, 73),

caractérisée par:

b) des moyens (27, 60, 70) de compression et d'égalisation légères de la matière moulue (11) dans la voie tubulaire de mesure (24, 25, 50, 105, 115),

c) un dispositif (12, 29, 31, 32, 33, 34, 45, 52, 72) de tassement supplémentaire de la matière moulue (11) précomprimée, perpendiculairement et vers le capteur de mesure (2, 30, 73) et

d) des moyens (35, 40) d'arrêt du déplacement de la matière moulue (11) ainsi tassée et de déclenchement de la phase proprement dite de mesure aux infrarouges.

2. Installation de mesure suivant la revendication 1, caractérisée en ce que les moyens de compression et d'égalisation légères de la matière moulue (11) sont des moyens de transport forcé (27, 60, 70).

3. Installation de mesure suivant la revendication 1 ou 2, caractérisée en ce qu'il est prévu un dispositif de commande (40) à l'aide duquel le transport forcé (27, 60, 70) peut être interrompu pendant la durée de la phase de mesure aux infrarouges.

4. Installation de mesure suivant l'une des revendications précédentes, caractérisée en ce qu'il est prévu des dispositifs indépendants de commande (35, 40) pour l'interruption du transport forcé, pour le tassement cyclique de la matière moulue et pour le déclenchement et la commande de la phase de mesure aux infrarouges.

5. Installation de mesure suivant l'une des revendications précédentes, caractérisée en ce que, dans la zone du capteur de mesure (30, 73), la voie de mesure (24, 25, 50, 105, 115) est agencée sous la forme d'un tube de mesure.

6. Installation de mesure suivant la revendication 5, caractérisée en ce que l'axe optique du capteur de mesure (30, 73) est orienté de manière sensiblement perpendiculaire à la surface de la matière moulue.

7. Installation de mesure suivant l'une des revendications précédentes, caractérisée en ce que le dispositif (29, 31, 32, 33, 34, 45, 52, 72) de tassement cyclique de la matière moulue comprend un élément de compression (32, 33, 34, 45, 52, 53, 72) qui peut se déplacer en direction du capteur de mesure (30, 73).

8. Installation de mesure suivant l'une des revendications précédentes, caractérisée en ce qu'il est associé à l'élément de compression (34, 52) un vérin pneumatique (31, 56) à course et pression réglables.

9. Installation de mesure suivant l'une des revendications 1 à 7, caractérisée en ce que l'élément de compression (34, 52) fait partie d'un électro-aimant à course et force réglables.

10. Installation de mesure suivant l'une des revendications 1 à 7, caractérisée en ce que l'élément de compression (52, 53) est une cuiller mobile (52).

11. Installation de mesure suivant la revendication 10, caractérisée en ce que la cuiller (52) est orientable et peut être déplacée vers le capteur de mesure (30), sensiblement suivant la direction de l'axe optique, à l'aide de moyens d'entraînement pneumatiques ou électriques.

12. Installation de mesure suivant la revendication 11, caractérisée en ce que la cuiller (52) est agencée sous une forme analogue à une cuiller à soupe, sa face concave, c'est-à-dire la face intérieure de cuiller, étant tournée vers le capteur de mesure (30), de sorte que la matière moulue peut être tassée sur le capteur de mesure (30) au moyen de cette cuiller (52).

13. Installation de mesure suivant l'une des revendications 1 à 7, caractérisée en ce que le dispositif de tassement cyclique de la matière moulue comprend des coussins gonflables de compression (45) qui sont disposés sur la voie de mesure (24) du côté opposé au capteur de mesure (30).

14. Installation de mesure suivant la revendication 13, caractérisée en ce que le coussin de compression (45) est disposé le long de la voie de mesure (24) et s'étend sur une longueur qui est un multiple de la dimension du capteur de mesure en longueur.

15. Installation de mesure suivant l'une des revendications 1 à 7, caractérisée en ce que le dispositif de tassement cyclique de la matière moulue est un vérin à manoeuvre électrique ou pneumatique (32).

16. Installation de mesure suivant l'une des revendications 115, caractérisée en ce que la voie de mesure (24) est une voie de mesure vibrée, le transport forcé s'effectuant à l'aide d'un vibrateur (27).

17. Installation de mesure suivant la revendication 16, caractérisée en ce que la voie de mesure vibrée est réalisée sous la forme d'une conduite de dérivation (21) d'une conduite principale (20).

18. Installation de mesure suivant la revendication 16 ou 17, caractérisée en ce que le vibrateur (27) possède une forme en cloche et est porté de façon suspendue à l'intérieur de la voie de mesure, ce vibrateur servant, d'une part, de moyen de transport forcé pour la voie de mesure (24), grâce à sa vibration, et formant, d'autre part, à l'extrémité inférieure de cette voie de mesure (24), un intervalle de dosage ("X") grâce à sa forme en cloche et à la partie associée de paroi d'une enveloppe (28).

19. Installation de mesure suivant l'une des revendications 16 à 18, caractérisée en ce que la voie de mesure (24) est agencée suivant le principe de trop-plein et est disposée dans une conduite de dérivation (21).

20. Installation de mesure suivant l'une des revendications 16 à 19, caractérisée en ce que le débit de dosage du transport par vibration est réglable de façon telle que le débit d'introduction dans la voie de mesure (24) est supérieur à l'extraction commandée due au transport par vibration, de sorte que la voie de mesure (24) est toujours pleine de matière moulue légèrement comprimée par la vibration.

21. Installation de mesure suivant l'une des revendications 1 à 15, caractérisée en ce que la voie de mesure (24) comprend un convoyeur à vis (70) disposé de préférence horizontalement et permettant le transport forcé et en ce qu'un élément (71) de retenue de la matière moulue est disposé à l'extrémité de cette voie de mesure (24).

22. Installation de mesure suivant la revendication 21, caractérisée en ce que l'élément de retenue (71) comprend un volet s'ouvrant sous la pression d'acheminement ou des fils ou lamelles mis en place à demeure.

23. Installation de mesure suivant la revendication 21 ou 22, caractérisée en ce que le dispositif (29, 31, 32, 33, 34, 45, 52, 72) de tassement cyclique de la matière moulue et le capteur de mesure (30, 73) sont l'un et l'autre disposés en amont du convoyeur à vis (60, 70) et/ou de l'élément de retenue (71) ou entre eux.

24. Procédé d'analyse quantitative continue de constituants se présentant sous forme de farine ou d'autres matières moulues pour produits alimentaires, selon lequel:

a) on dirige la matière moulue (11) à l'aide d'une voie de mesure aux infrarouges (24, 25, 50, 105, 115) et on l'y tasse,

b) on soumet la matière moulue (11) tassée à un rayonnement de lumière infrarouge et, à partir des rayons que l'échantillon de mesure réfléchit, en les diffusant, vers le capteur de mesure (2, 30, 73) et à l'aide d'un dispositif de mesure (29, 31, 32, 33, 34, 45, 52, 72), on détermine la teneur en protéines, la teneur en eau, la teneur en cendres et/ou la chromaticité de cet échantillon de mesure,

caractérisé en ce que:

c) en premier lieu, on comprime légèrement et on égalise légèrement la matière moulue (11) dans la zone du capteur de mesure (2, 30, 73),

d) en la maintenant immobile, on soumet la matière moulue (11) légèrement comprimée et égalisée à un tassement supplémentaire suivant une direction perpendiculaire au capteur de mesure (2, 30, 73) et vers celui-ci et

e) à l'aide d'un premier ordinateur, on traite les signaux de mesure obtenus à partir de l'échantillon de mesure ainsi tassé afin d'obtenir des valeurs quantitatives de mesure.

25. Procédé suivant la revendication 24, caractérisé en ce qu'on réalise la compression et l'égalisation légères de la matière moulue (11) au cours d'un transport forcé.

26. Procédé suivant la revendication 24 ou 25, caractérisé en ce que, sur la base de valeurs préfixées mises en mémoire et à l'aide d'un second ordinateur, on utilise directement les valeurs de mesure obtenues pour commander automatiquement le mélange des matières brutes et/ou l'addition d'eau et/ou le mélange de farines, en réalisant la commande et la régulation du moulin en ce qui concerne la teneur de la matière moulue en protéines et/ou en eau.

27. Procédé suivant l'une des revendications 24 à 26, caractérisé en ce que, pour exécuter une phase de mesure, on assure une légère compression de la matière moulue, ou le maintien de l'absence de vides dans celle-ci, en mettant à l'arrêt ou en interrompant le transport forcé, on prépare l'échantillon de mesure en tassant cette matière moulue par des moyens mécaniques dans la zone du capteur de mesure et on conserve en l'état cet échantillon de mesure tassé afin d'en mesurer les éléments constitutifs, par exemple les protéines, l'eau, les cendres et la blondeur.

28. Procédé suivant l'une des revendications 24 à 26, caractérisé en ce qu'on répète les phases de mesure de manière cyclique, à intervalles de temps pouvant être choisis.

29. Procédé suivant l'une des revendications 24 à 26, caractérisé en ce qu'on répète éventuellement chaque mesure individuelle pendant une phase de mesure, on fait la moyenne des résultats de mesure d'un groupe de phases de mesure et les fournit au second ordinateur, en tant que valeurs effectives, afin de commander le mélange des matières brutes et/ou la teneur en eau et/ou le mélange des farines et on procède à la régulation des paramètres du produit sur des valeurs préfixées déterminées en protéines et en eau.

30. Procédé suivant la revendication 24, caractérisé en ce que, lors de chaque exécution d'une phase de mesure, le tassement de la matière moulue, alors non déplacée, est maintenu sensiblement constant pendant la durée de la mesure.

31. Procédé suivant l'une des revendications 24 à 30, caractérisé en ce que, pour le tassement mécanique de l'échantillon de mesure, on utilise en supplément un élément élastique par nature ou par structure.

32. Procédé suivant l'une des revendications 24 à 31, caractérisé en ce que, pendant une phase de mesure, on procède à plusieurs mesures pour chaque domaine différent du spectre et, à partir des multiples résultats de mesure, on forme une valeur moyenne qu'on compare alors à la valeur de référence correspondant au domaine considéré du spectre.

33. Procédé suivant l'une des revendications 24 à 32, caractérisé en ce qu'on réalise la moyenne des résultats de mesure à partir d'un certain nombre de phases de mesure différentes et on applique la valeur moyenne ainsi calculée, en tant que valeur effective, sur un autre ordinateur.

34. Procédé de surveillance des constituants, c'est-à-dire des éléments constitutifs, de produits alimentaires du type farine ou de matières moulues pour produits alimentaires, selon lequel:

a) on dirige de façon continue la matière moulue à l'aide d'une voie de mesure (24, 25, 50, 105, 115) et on l'y tasse,

b) d'une manière continue et successive, on soumet la matière moulue tassée à un rayonnement de lumière infrarouge et on détermine la proportion des différents constituants en mesurant l'intensité de la lumière réfléchie par la matière moulue dans les domaines du spectre associés à ces différents constituants,

caractérisé en ce que:

c) en premier lieu, on comprime légèrement, dans la voie de mesure (24, 25, 50, 105, 115), la matière moulue destinée aux mesures réalisées de manière continue et successive et on en égalise la surface,

d) puis on procède à un tassement supplémentaire de la matière moulue précomprimée, perpendiculairement au capteur de mesure (2, 30, 73) et vers celui-ci,

e) pendant chaque mesure, on interrompt le déplacement de l'échantillon de mesure (11) à l'intérieur de la voie de mesure aux infrarouges (24, 25, 50, 105, 115),

f) on se sert d'un premier ordinateur pour établir les valeurs des proportions des constituants et on les compare chacune à des valeurs de consigne préfixées par un second ordinateur et,

g) en fonction des écarts déterminés entre valeurs établies et valeurs préfixées, on prend des dispositions sur le plan commande, par exemple pour le mélange des matières brutes, la combinaison des farines, l'addition d'eau, l'adjonction de gluten, les valeurs de référence pour les réglages des moulins.

Fig. 1

Fig. 2

Fig. 3

EP 0 179 108 B1

Fig. 4

Fig.5

Drucker
-Schreiber

36

40

Mikrocomputer

35

Minicomputer

39

Elektronik

38

Optik

30

V ▾ ⊻

v ⊻

23

21

20

25

32

31  29

34

33

37

X

28

V

27

26

24

22

Fig.5a

Fig.6

Fig.6a

Fig.7

Fig. 8

11

Fig.9

Fig.10

Fig.11A

EP 0 179 108 B1

Fig.11B

Fig.12

90,90'

91

92

93

94

95

96

97

A    A    A

EP 0 179 108 B1